# EUROPEAN PATENT APPLICATION

(11) **EP 4 169 524 A1**
(43) Date of publication of application: **26.04.2023**
(21) Application number: 21204596.7
(22) Date of filing: 25.10.2021
(51) Int. Cl.: A61K 38/20, A61K 38/21, A61K 35/17, A61P 19/02, A61F 7/02, A61K 9/06

(54) **IMMUNOMODULATORY SUBSTANCE FOR USE IN A METHOD FOR TREATMENT AND/OR PREVENTION OF A DISEASE**

(71) Applicant: Ellennbe GmbH, 83670 Bad Heilbrunn (DE)
(72) Inventor: Neumann, Lydia Ellen, 83670 Bad Heilbrunn (DE)
(74) Representative: Kador & Partner Part mbB

(57) **Abstract**

The present invention relates to an immunomodulatory substance for use in a method for treating and/or preventing an inflammatory disease, immunological disease and/or autoimmunological disease, wherein the method comprises the steps of:
(A) generating an accumulation of PBMCs within the skin of a subject; and
(B) administering the immunomodulatory substance to the skin of said subject.

The present invention further relates to a method for treating and/or preventing an inflammatory disease, immunological disease and/or autoimmunological disease, wherein the method comprises the steps of:
(A) generating an accumulation of PBMCs within the skin of a subject; and
(B) administering the immunomodulatory substance to the skin of said subject.

The present invention further relates to a topical dosage form comprising an immunomodulatory substance; and/or a dosage form suitable for injection comprising an immunomodulatory substance;

## Description

The present invention relates to an immunomodulatory substance for use in a method for treating and/or preventing diseases in a subject, such as inflammatory diseases, immunological diseases and autoimmunological diseases.

The invention further relates to compositions and to kits of parts comprising an immunomodulatory substance as an active ingredient, in particular for use in the prevention and/or treatment of diseases, such as inflammatory diseases, immunological diseases and autoimmunological diseases.

The invention still further relates to methods of preventing and/or treating diseases, such as inflammatory diseases, immunological diseases and autoimmunological diseases in a subject.

Inflammatory diseases, immunological diseases and autoimmunological diseases that cause damaging and painful inflammatory reactions with severe impact on life quality are widespread diseases which affect huge parts of the human and animal population. In particular, chronic course of such diseases can cause extreme suffering over long periods of time for the concerned subjects.

The methods known for treatment and prevention of such diseases are still unsatisfactory to a large degree. For example, glucocorticoids are used for treatment of these diseases, however, besides their beneficial effects in reducing inflammation, they have considerable negative side effects, especially in long-term use.

On the other hand, immunotherapies may be available which, however, require immense personal und material resources and can only be provided in specialized health care centers. Hence, such therapies are unsuitable for a broad public application.

It would therefore be highly desirable to provide alternative agents and methods for treatment and prevention of inflammatory diseases, immunological diseases and autoimmunological diseases, which, accordingly, was the object of the present invention.

Highly surprisingly, it was found that this object can be achieved by use of an immunomodulatory substance in a method where it is brought in contact with peripheral blood mononuclear cells (PBMCs) in vivo in a subjects body part such as the skin.

In a first step of this method, PBMCs, particularly immune cells like lymphocytes, more particularly naïve lymphocytes like naïve T-cells, are attracted to accumulate e.g. within the skin by, for example, enhancing blood circulation and/or blood vessel dilation in the skin, and in a second step the immunomodulatory substance is administered in order to provide immunomodulatory modulated PBMCs within e.g. the skin tissue.

Hence, the present invention relates to an immunomodulatory substance for use in a method for treating and/or preventing an inflammatory diseases, immunological disease and/or autoimmunological disease, wherein the method comprises the steps of:
(A) generating an accumulation of PBMCs within the skin of a subject; and
(B) administering the immunomodulatory substance to the skin of said subject; and to said method as such.

Administering the immunomodulatory substance to the skin of a subject in step (B) may be carried out by administering the immunomodulatory substance as such or as a component of a composition, i.e. as a composition comprising the immunomodulatory substance.

This applies generally in the present invention and hence applies to any one of the embodiments of the invention described herein.

Furthermore, administering the immunomodulatory substance to the skin of a subject in step (B) may involve to administer the immunomodulatory substance in a topical dosage form comprising the immunomodulatory substance, and/or to administer the immunomodulatory substance in a dosage form suitable for injection comprising the immunomodulatory substance.

This also applies generally in the present invention and hence applies to any one of the embodiments of the invention described herein.

The new medical use of the present invention provides an entirely new principle for treatment and prevention of inflammatory diseases, immunological diseases and autoimmunological diseases, and has a multitude of advantages for the subjects treated.

First, the new medical use is very effective in the treatment and/or prevention of inflammatory diseases, immunological diseases and autoimmunological diseases and has no adverse side effects. Thus, the new medical use may also contribute to mental and/or physical wellbeing of the treated subjects.

The use is easy to perform, for example, because both steps (A) and (B) are performed on or within the skin. The skin has the advantage to be easily accessible for a treatment, injections and/or treatments into the skin are generally of lower health risk for a subject like for instance intravenous injections and the skin tissue provide a tight structure with little blood circulation.

The subjects may in several embodiments of the new use self-apply the method steps of the use, and the costs for the use are low.

Still further, there is no risk of contamination or swopping as body identical substances are used.

Without wishing to be bound be theory, it is believed that the new use is based on the following principles: The blood supply of the sub-topical skin layers and particularly the dermis is essentially provided by capillary vessels. In contrast to veins and arteries, capillaries have a very narrow cross section. As a consequence, PBMCs, particularly naïve T-cells, are not present or at least not in effective amounts within the skin capillaries and hence within the skin, simply, because they are too big to squeeze through non-dilated capillaries. Solely erythrocytes and thrombocytes, which are deprived of a nucleus, are compressible and flexible enough to enter and stream through non-dilated capillaries in order ensure the skin's oxygen supply and skin integrity.

Hence, in order to generate a temporal accumulation of PBMCs within the skin tissue, they first must be given access to the capillaries to bring them in the vicinity of the skin tissue. This is accomplished by step (A) by generating an accumulation of PBMCs within the skin of a subject, in particular within the lumen of the capillaries of the skin.

In a second step, the PBMCs must be caused to leave the capillaries and to migrate into the adjacent skin tissue. After migration into the skin tissue, the PBMCs unlike in the blood are trapped in the skin tissue and cannot easily be flushed away. The temporal trapping allows to incubate the PBMCs, such as lymphocytes, more particularly naïve lymphocytes like naïve T-cells, for a time sufficient for maturation, in case respective immunomodulatory substances like cytokines are provided within the skin. After maturation, the matured cells re-enter the blood stream and migrate to their place of action.

The one or more immunomodulatory substances, in particular IFN-γ, thereby take a double function. Firstly, they function as the attractor for the PBMCs, such as lymphocytes, in particular naïve T-cells, to start rolling to a complete stop along the capillary walls in order to subsequently cross through the capillary wall and migrate into the skin tissue. Secondly, the one or more immunomodulatory substances, particularly IFN-γ contribute to the desired maturation of the lymphocytes. In case of IFN-γ, the PBMCs mature in the presence of IFN-γ and dentritic cells, which inherently present within the skin, and preferably, in simultaneous absence of an immune challenge and/or immune activity towards inflammation-suppressive regulatory T-cells.

Hence, the combined effect of step (A) and (B) is the generation of an accumulation of PBMCs, within the skin tissue and the maturing of such cells.

In case of an injection of PMBCs, the effect of generating a population of lymphocytes achieve by steps (A) and (B) within the skin tissue is accomplished by the injection of the PBMCs into the skin tissue.

### DEFINITIONS AND PREFERRED EMBODIMENTS

The following definitions and preferred embodiments relate generally to any one of the embodiments of the present invention as described herein even of not explicitly mentioned, unless described otherwise.

The expression "*PBMCs*" ("*peripheral blood mononuclear cells*") refers to a sub-population of cells of the cellular blood components, particularly of the leucocytes. Usually, PBMCs comprise, preferably consist of, lymphocytes and monocytes, whereas erythrocytes and platelets (do not possess a nucleus) and granulocytes (possess multi-lobed nuclei) are absent or essentially absent.

PBMCs may be extracted from the blood, preferably whole blood, and may then be present PBMCs in isolated, preferably purified, form, in the following termed *"isolated PBMCs",* or they may be present as a sub-population of cells of the cellular blood components within the blood, preferably whole blood, in the following termed *"blood PBMCs".* In isolated PBMCs, blood components and cellular blood components other than PBMCs are absent or essentially absent. Isolated PBMCs may be obtained by any method known to the person skilled in the art, preferably by ficoll-extraction in combination with gradient centrifugation or by apharesis, more preferably by apharesis, using whole blood.

The expression *"lymphocytes"* refer to a sub-population of PBMCs. Usually, lymphocytes comprise, preferably consist of, B-cells including naïve and mature B-cells, natural killer cells and/or T-cells including naïve and mature T-cells.

Preferably, the lymphocytes are naïve lymphocytes, preferably comprising, more preferably consisting of, naïve B-cells and naïve T-cells. More preferably, the lymphocytes are naïve T-cells.

The expression "*naïve T-cells"* refers to T-cells that have differentiated in the thymus, and successfully undergone the positive and negative processes of central selection in the thymus. Naïve T-cells have the potential to mature to different mature types of T-cells like helper T-cells, cytotoxic T-cells and/or regulatory T-cells.

Preferably, the PBMCs in any one of the embodiments of the invention as described herein are lymphocytes, such as T-cells, more preferably are naïve lymphocytes, and most preferably are naïve T-cells.

The definitions of PBMCs and the preferred embodiments thereof apply generally to any one of the embodiments of the present invention wherein PBMCs, lymphocytes, naïve lymphocytes, T-cells and/or naïve T-cells are mentioned.

The disease to be treated and/or prevented can be any inflammatory diseases, immunological diseases and/or autoimmunological diseases.

It may be pointed out that an inflammatory diseases may have an immunological background or an autoimmunological background, while an immunological disease or autoimmunological disease must not necessarily be associated with an inflammation.

More preferably, the inflammatory diseases comprise, preferably consist of, allergic rhinitis (hay fever), Multiple sclerosis, diabetic diseases, arthritis and/or synovitis and/or inflammatory rheumatic diseases.

Preferably, the arthritis comprises, preferably consists of, monoarthritis, oligoarthritis and/or polyarthritis, more preferably is a polyarthritis, preferably M13.-, M14.-, M15.- and/or M25.-, more preferably M25.5 in accordance with the standard ICD-10-GM 2021.

Preferably, the synovitis comprise, preferably consist of, synovitis and tenosynovitis, preferably M65.- in accordance with the standard ICD-10-GM 2021 ("ICD-10-GM 2021 Systematisches Verzeichnis", Version 2021, Deutscher Ärzteverlag, ISBN 978-3-7691-3722-4).

Preferably, the inflammatory rheumatic diseases comprise, preferably consists of:
- rheumatoid arthritis (RA) (also called chronic polyarthritis), preferably M05.-, more preferably M05.80 in accordance with the standard ICD-10-GM 2021;
- juvenile arthritis, preferably M08.- in accordance with the standard ICD-10-GM 2021;
- spondyloarthritides including Morbus Bechterew (BD) (also called ankylosing spondylitis) preferably M45.-, in accordance with the standard ICD-10-GM 2021, psoriatic arthritis preferably M07.- in accordance with the standard ICD-10-GM 2021, enteropathic arthritis (associated with intestinal diseases such as ulcerative colitis) preferably M07.- in accordance with the standard ICD-10-GM 2021, reactive arthritis preferably M02.- in accordance with the standard ICD-10-GM 2021, and undifferentiated spondyloarthritis;
- collagenoses (connective tissue diseases) like, systemic sclerosis, sjögren's syndrome, polymyositis, dermatomyositis and mixed collagenosis, preferably M30-M36 in accordance with the standard ICD-10-GM 2021, and like lupus erythematosus, preferably L93.- in accordance with the standard ICD-10-GM 2021;
- vasculitides (diseases with vascular inflammation), preferably L93.-, L95.- or M05.- in accordance with the standard ICD-10-GM 2021; and/or
- polymyalgia rheumatic, preferably M35.3 in accordance with the standard ICD-10-GM 2021.

More preferably, the inflammatory rheumatic diseases are rheumatoid arthritis (RA) and/or Morbus Bechterew (BD).

More preferably the inflammatory diseases comprise, preferably consist of, polyarthritis, rheumatoid arthritis (RA) and/or Morbus Bechterew (BD).

The arthritis, monoarthritis, oligoarthritis, polyarthritis and/or any of the inflammatory rheumatic diseases listed above may be, preferably, is defined and/or determined by any method(s) and/or parameter(s) commonly used in the art and known to the person skilled in the art. Preferably, the definition and/or determination is in accordance with the standard ICD-10-GM 2021.

More preferably, the definition and/or determination of the polyarthritis is in accordance with the standard ICD-10-GM 2021, preferably M13.-, M14.-, M15.- and/or M25.-, more preferably M25.5 in accordance with the standard ICD-10-GM 2021.

More preferably, the definition and/or determination of the rheumatoid arthritis is in accordance with the standard ICD-10-GM 2021, preferably M05.-, more preferably M05.80 in accordance with the standard ICD-10-GM 2021.

More preferably, the definition and/or determination of the Morbus Bechterew is in accordance with the standard ICD-10-GM 2021, preferably M45 in accordance with the standard ICD-10-GM 2021.

The immunomodulatory substance as used in the present invention must be/is capable of affecting the PBMCs.

More specifically, the immunomodulatory substance is capable of regulating, inducing, supressing, maturing, differentiating, proliferating, modulating and/or balancing the amounts PBMCs. The immunomodulatory substance may be, preferably is, any type of substance, similar or identical to any protein or fragment thereof naturally occurring in a vertebrate, preferably a mammal, more preferably a subject as defined below. More preferably, the immunomodulatory substance is a cytokine, still more preferably an immune-related cytokines.

More preferably, the immunomodulatory substance is a cytokine regulating, inducing, maturing, differentiating, proliferating, modulating and/or balancing the amounts of the PBMCs, preferably of lymphocytes, particularly naïve lymphocytes, more preferably of T-cells particularly naïve T-cells, and/or balancing the amounts of different PBMCs, preferably of lymphocytes, particularly naïve lymphocytes, more preferably of T-cells particularly naïve T-cells.

Even more preferably, the immunomodulatory substance is selected from the group of interferons, interleukins, colony-stimulating factors, tumour necrosis factors and/or chemokines. Still more preferably, the immunomodulatory substance is selected from the group of interferons and/or interleukins, most preferably IFN-γ (interferon gamma) and/or IL-2 (interleukin 2), most preferably IFN-γ.

Hence, in a particularly preferred embodiment the invention relates to an immunomodulatory substance for use in a method for treating and/or preventing an inflammatory diseases, immunological diseases and autoimmunological diseases, wherein the method comprises the steps of:
(A)generating an accumulation of PBMCs within the skin of a subject; and
(B) administering IFN-γ to the skin of said subject;
and to said method as such.

Administering the immunomodulatory substance to the skin of a subject may be carried out by administering the immunomodulatory substance as such or as a component of a composition, i.e. as a composition comprising the immunomodulatory substance.

Furthermore, administering the immunomodulatory substance to the skin of a subject may involve to administer the immunomodulatory substance in a topical dosage form comprising the immunomodulatory substance, and/or to administer the immunomodulatory substance in a dosage form suitable for injection comprising the immunomodulatory substance.

The subject may be, preferably, is any subject in need of the treatment and/or prevention. Preferably, the subject is a subject being diagnosed with the inflammatory diseases, immunological diseases and autoimmunological diseasess and/or a subject for which it is indicative to be at risk to develop the inflammatory diseases, immunological diseases and autoimmunological diseases.

Usually, in a subject being diagnosed with the inflammatory diseases, immunological diseases and autoimmunological diseases, the symptoms of the inflammatory diseases, immunological diseases and autoimmunological diseases are already apparent and the subject suffers from the symptoms of the inflammatory diseases, immunological diseases and autoimmunological diseases. In other words, the inflammatory diseases, immunological diseases and autoimmunological diseases have already been broken out in the subject.

Usually, in a subject for which it is indicative to be at risk to develop the inflammatory diseases, immunological diseases and autoimmunological diseases, the symptoms of the inflammatory diseases, immunological diseases and autoimmunological diseases are not yet apparent and the subject does not suffer from the symptoms of the inflammatory diseases, immunological diseases and autoimmunological diseases. In other words, the inflammatory diseases, immunological diseases and autoimmunological diseases have not yet been broken out in the subject. To be at risk is to be understood in the sense that the subject will develop and/or will develop with a high chance the inflammatory diseases, immunological diseases and autoimmunological diseases.

The diagnosis of the subject being diagnosed with the inflammatory diseases, immunological diseases and autoimmunological diseases may be, preferably, is established by any method(s) and/or parameter(s) commonly used in the art and known to the person skilled in the art. Preferably, the diagnosis is established in accordance with the standard ICD-10-GM 2021.

Preferably, the subject being diagnosed with the inflammatory diseases, immunological diseases and autoimmunological diseases is a subject diagnosed with arthritis, monoarthritis, oligoarthritis, polyarthritis and/or any of the inflammatory rheumatic diseases listed above.

More preferably, the diagnosis of the subject being diagnosed with polyarthritis is established in accordance with the standard ICD-10-GM 2021, preferably M13.-, M14.-, M15.- and/or M25.-, more preferably M25.5 in accordance with the standard ICD-10-GM 2021.

More preferably, the diagnosis of the subject being diagnosed with rheumatoid arthritis is established in accordance with the standard ICD-10-GM 2021, preferably M05.-, more preferably M05.80 in accordance with the standard ICD-10-GM 2021.

More preferably, the diagnosis of the subject being diagnosed with Morbus Bechterew is established in accordance with the standard ICD-10-GM 2021, preferably M45.-, in accordance with the standard ICD-10-GM 2021.

The indication of the subject for which it is indicative to be at risk to develop the inflammatory diseases, immunological diseases and autoimmunological diseases may be, preferably, is established by any method(s) and/or parameter(s) commonly used in the art and known to the person skilled in the art. More preferably, the indication is established in accordance with the ICD-10-GM 2021.

The indication of the subject for which it is indicative to be at risk to develop the inflammatory diseases, immunological diseases and autoimmunological diseases may be, preferably, is established by any method(s) and/or parameter(s) commonly used in the art and known to the person skilled in the art, for instance family history and/or genetic association like HLA-B*27 in case of Morbus Bechterew.

Preferably, the subject for which it is indicative to be at risk to develop the inflammatory diseases, immunological diseases and autoimmunological diseases, is a subject for which it is indicative to develop arthritis, monoarthritis, oligoarthritis, polyarthritis and/or any of the inflammatory rheumatic diseases listed above.

Preferably, the expression *"treating"* refers to the subject being diagnosed with the inflammatory diseases, immunological diseases and autoimmunological diseases. Thereby, such subject's condition is improved e.g. pain and/or inflammation are relieved or disappear, joint destruction is prevented, further progress of joint destruction is slowed down, stopped and/or regeneration has occurred, the joints functional ability is preserved or improved, the subject's normal lifestyle maintained, the subject's quality of life and/or mental and/or physical wellbeing are maintained or improved.

Preferably, the expression *"preventing"* refers to the subject being diagnosed to be at risk to develop the inflammatory diseases, immunological diseases and autoimmunological diseases. Thereby, the subject's healthy condition is maintained and the onset of the symptoms of the inflammatory diseases, immunological diseases and autoimmunological diseasess in the subject is prevented and/or delayed, joint destruction is prevented, the subject's normal lifestyle maintained, the subject's quality of life and/or mental and/or physical wellbeing are maintained or improved.

Furthermore, the subject may be, preferably, is any subject having an immune system capable of rising an adaptive immune response, preferably, capable of rising a T-cell immune response.

Preferably, the subject is a vertebrate, more preferably a mammal, even more preferably any of the genus and/or species human or a mammal animal selected from cow, buffalo, horse, donkey, elephant, sheep, goat, pig, rabbit, mouse, rat, camel, dromedary, lama, alpaca, dog or cat. Still more preferably the subject is a human.

Furthermore, the subject may be, preferably, is a newborn, suckling, infant, child, adolescent and/or adult of the subject, preferably a suckling, infant, child, adolescent and/or adult, still more preferably an infant, child, adolescent or adult, still more preferably a child, adolescent and/or adult, still even more preferably an adolescent and/or adult and most preferably an adult. In case the subject is a human, a newborn is until 28^{th} day of life, a suckling from the beginning of the 29^{th} day of life until the end of the 12^{th} month of life, an infant from the beginning of the 13^{th} month to the completed 3^{rd} year of life, a child from the beginning of the 4^{th} to the completed 12^{th} year of life, an adolescent from the beginning of the 13^{th} to the completed 18^{th} year of life and an adult from the beginning of the 19^{th} year of life.

In an aspect of all embodiments of the invention, the method further may comprise the step of:
(C)administering a second immunomodulatory substance to the skin of the subject.

The second immunomodulatory substance administered in step (C) is different from said first immunomodulatory substance administered in step (B).

The second immunomodulatory substance administered in step (C) may be selected from any one of the embodiments of the immunomodulatory substance as described herein. In a particularly preferred embodiment, the second immunomodulatory substance administered in step (C) comprises or consists of an interleukin, more preferably IL-2.

Administering the second immunomodulatory substance to the skin of a subject in step (C) may be carried out by administering the immunomodulatory substance as such or as a component of a composition, i.e. as a composition comprising the immunomodulatory substance.

Such a composition may also comprise both immunomodulatory substances to be administered in steps (B) and (C).

Furthermore, administering the immunomodulatory substance to the skin of a subject in step (C) may involve to administer the immunomodulatory substance in a topical dosage form comprising the immunomodulatory substance, and/or to administer the immunomodulatory substance in a dosage form suitable for injection comprising the immunomodulatory substance.

Such a topical dosage form and/or dosage form suitable for injection may also comprise both immunomodulatory substances to be administered in steps (B) and (C).

This applies to any one of the embodiments of the invention described herein involving steps (B) and (C).

Preferably, the immunomodulatory substances to be administered in (C) comprises, or consists of, IL-2.

IL-2 has the technical effect of especially synergistically prolonging, enhancing and/or boosting the technical effect of IFN-γ.

In all embodiments as described herein wherein a first and a second immunomodulatory substance are administered to the skin of a subject in steps (B) and (C), respectively, it is preferred that the first immunomodulatory substance is selected from any one of the above described embodiments, more preferably is selected from the group of interferons, and the second immunomodulatory substance is selected from any one of the above described embodiments, more preferably is selected from the group of interleukins.

Most preferably, the first immunomodulatory substance is IFN-γ and the second immunomodulatory substance is IL-2.

Hence, still more preferably, the present invention relates to:
- IFN-γ;
- IL-2;
- a combination of the IFN-γ and IL-2;
- a composition comprising the IFN-γ;
- a composition comprising the IL-2;
- a composition comprising the IFN-γ and IL-2;
- a kit of parts comprising IFN-γ;
- a kit of parts comprising IFN-γ; and/or
- a kit of parts comprising IFN-γ and IL-2,
for use in a method for treating inflammatory diseases, wherein the method comprises the steps of:
(A) generating an accumulation of PBMCs within a skin of a subject;
(B) administering the IFN-γ to the skin of said subject,
   preferably by administering the IFN-γ, the composition comprising IFN-γ and/or the composition comprising IFN-γ and IL-2 to the skin; and
(C) administering the IL-2 to the skin of said subject,
   preferably by administering the IL-2, the composition comprising IL-2 and/or the composition comprising IFN-γ and IL-2 to the skin.

Still more preferably, the present invention relates to;
- a topical dosage form comprising IFN-γ;
- a topical dosage form comprising IL-2;
- a topical dosage form comprising IFN-γ and IL-2; and/or
- a dosage form suitable for injection comprising IFN-γ,
- a dosage form suitable for injection comprising IL-2
- a dosage form suitable for injection comprising IFN-γ and IL-2;
in a method for treating and/or preventing inflammatory diseases, wherein the method comprises the steps of:
(A) generating an accumulation of PBMCs within the skin of a subject;
(B) administering the IFN-γ to the skin of said subject,
   preferably by applying the topical dosage form comprising IFN-γ and/or the topical dosage form comprising IFN-γ and IL-2 to the skin and/or by performing an injection by using the dosage form suitable for injection comprising IFN-γ and/or comprising IFN-γ and IL-2; and
(C) administering the IL-2 to the skin of said subject ,
   preferably by applying the topical dosage form comprising IL-2 and/or the topical dosage form comprising IFN-γ and IL-2 to the skin and/or by performing an injection by using the dosage form suitable for injection comprising IL-2 and/or comprising IFN-γ and IL-2.

The IFN-γ (interferon gamma) may be, preferably is, any IFN-γ, IFN-γ-analogon or fragment of these as long as it has a biologic activity of IFN-γ, preferably, in the subject. More preferably, the biologic activity is sufficient in order to achieve the treating and/or preventing of the inflammatory diseases, immunological diseases and autoimmunological diseasess, preferably, in the subject. The biologic activity is preferably indicated in international units (IU). Hence, the IFN-γ does not necessarily be derived from or be identical to the IFN-γ of the same genus and/or species the subject belongs to as long as it has a sufficient biologic activity, i.e. cross-reactivity, in the subject.

More preferably, the IFN-γ is identical to an/the IFN-γ of the same genus and/or species the subject belongs to, this preferably means that the IFN-γ is in respect to its primary, secondary, tertiary and quaternary protein structure identical to an/the IFN-γ of the same genus and/or species the subject belongs to, more preferably, also in view of modifications, particularly glycosylations.

Still more preferably, the IFN-γ is an IFN-γ, which is derived from an individual belonging to the same genus and/or species as the subject, or which is identical to an/the IFN-γ of the same genus and/or species the subject belongs to. For instance:
in case the subject is a human the IFN-γ is human IFN-γ;
in case the subject is a cow the IFN-γ is bovine IFN-γ;
in case the subject is a horse the IFN-γ is equine IFN-γ;
in case the subject is a donkey the IFN-γ is donkey IFN-γ;
in case the subject is an elephant the IFN-γ is elephant IFN-γ;
in case the subject is a sheep the IFN-γ is sheep IFN-γ;
in case the subject is a goat the IFN-γ is goat IFN-γ;
in case the subject is a pig the IFN-γ is porcine IFN-γ;
in case the subject is a rabbit the IFN-γ is rabbit IFN-γ;
in case the subject is a mouse the IFN-γ is mouse IFN-γ;
in case the subject is a rat the IFN-γ is rat IFN-γ;
in case the subject is a camel the IFN-γ is camel IFN-γ;
in case the subject is a dromedary the IFN-γ is dromedary IFN-γ;
in case the subject is a lama the IFN-γ is lama IFN-γ;
in case the subject is a alpaca the IFN-γ is alpaca IFN-γ;
in case the subject is a dog the IFN-γ is dog IFN-γ; and/or
in case the subject is a cat the IFN-γ is cat IFN-γ.

Preferably, the IFN-γ is not the subject's endogenous IFN-γ. In other words, the IFN-γ for use/used in a method according to the present invention is not an IFN-γ isolated from the subject's body.

Furthermore, the IFN-γ may be, preferably, is a recombinant IFN-γ generated by any recombinant expression techniques known to the person skilled in the art, a chemically synthesized IFN-γ synthesized by any production techniques known to the person skilled in the art, a naturally occurring IFN-γ obtained from natural sources or any combination thereof. Preferably, the IFN-γ is recombinant IFN-γ, chemically synthesized IFN-γ or any combination thereof, most preferably recombinant IFN-γ, even more preferably recombinant human IFN-γ-1b (interferon gamma-1b) preferably produced in genetically modified *Escherichia coli* (produced by Boehringer Ingelheim RCV GmbH & Co KG and in Germany commercially available from Boehringer Ingelheim Pharma GmbH & Co. KG under the tradename Imukin^{®}).

The IL-2 (interleukin 2) may be, preferably, is any IL-2, IL-2-analogon or fragment of these as long as it has a biologic activity of IL-2, preferably, in the subject. More preferably, the biologic activity is sufficient in order to achieve the treating and/or preventing of the inflammatory diseases, immunological diseases and autoimmunological diseasess, preferably, in the subject. The biologic activity is preferably indicated in international units (IU). Hence, the IL-2 does not necessarily be derived from or be identical to the IL-2 of the same genus and/or species the subjects belongs to as long as it has a sufficient biologic activity, i.e. cross-reactivity, in the subject.

More preferably, the IL-2 is identical to an/the IL-2 of the same genus and/or species the subject belongs to, this means that the IL-2 is in respect to its primary, secondary, tertiary and quaternary protein structure identical to an/the IL-2 of the same genus and/or species the subject belongs to, more preferably, also in view of modifications, particularly glycosylations.

Still more preferably, the IL-2 is an IL-2, which is derived from an individual belonging to the same genus and/or species as the subject, or which is identical to an/the IL-2 of the same genus and/or species the subject belongs to. For instance:
in case the subject is a human the IL-2 is human IL-2;
in case the subject is a cow the IL-2 is bovine IL-2;
in case the subject is a horse the IL-2 is equine IL-2;
in case the subject is a donkey the IL-2 is donkey IL-2;
in case the subject is an elephant the IL-2 is elephant IL-2;
in case the subject is a sheep the IL-2 is sheep IL-2;
in case the subject is a goat the IL-2 is goat IL-2;
in case the subject is a pig the IL-2 is porcine IL-2;
in case the subject is a rabbit the IL-2 is rabbit IL-2;
in case the subject is a mouse the IL-2 is mouse IL-2;
in case the subject is a rat the IL-2 is rat IL-2;
in case the subject is a camel the IL-2 is camel IL-2;
in case the subject is a dromedary the IL-2 is dromedary IL-2;
in case the subject is a lama the IL-2 is lama IL-2;
in case the subject is a alpaca the IL-2 is alpaca IL-2;
in case the subject is a dog the IL-2 is dog IL-2; and/or
in case the subject is a cat the IL-2 is cat IL-2.

Preferably, the IL-2 is not the subject's endogenous IL-2. In other words, the IL-2 for use in the method/medical use according to the present invention is not an IL-2 isolated from the subject's body.

Furthermore, the IL-2 may be, preferably, is a recombinant IL-2 generated by any recombinant expression techniques known to the person skilled in the art, a chemically synthesized IL-2 synthesized by any production techniques known to the person skilled in the art, a naturally occurring IL-2 obtained from natural sources or any combination thereof. Preferably, the IL-2 is recombinant IL-2, chemically synthesized IL-2 or any combination thereof, most preferably recombinant IL-2, even more preferably recombinant human IL-2, most preferably Aldesleukin, preferably produced in genetically modified *Escherichia coli* (produced by Novartis Pharmaceuticals UK, Limited and in Germany commercially available from Novartis Pharma GmbH under the tradename Proleukin^{®}S).

Preferably, the skin comprises, preferably consists of, in skin thickness direction of the skin surface and one or more skin layers. The one or more skin layers comprise, preferably consist of, epidermis, comprising stratum disjunctum, stratum corneum, stratum lucidum, stratum granulosum, stratum spinosum and stratum basale; dermis; and subcutis.

The expression *"skin thickness direction"* as used in the present invention refers to the direction perpendicular to the skin's surface.

The expression *"underneath",* as used in the present invention, when used with reference to the skin or skin layers, defines a position located closer to the body centre and hence further away from the skin surface.

The expression *"above",* as used in the present invention, when used with reference to the skin or skin layers, defines a position located further away from the body centre and hence closer to the skin surface.

The expression *"sub-topical layers"* of the skin as used in the present invention refers to the layers of the skin comprising, preferably consisting of, stratum spinosum, stratum basale, dermis and subcutis. More preferably, comprising, even more preferably consisting of, dermis and subcutis.

The expression *"skin tissue"* as used in the present invention refers to the skin excluding any blood vessels like capillaries and their vessel/capillary lumen. For instance, the expression *"skin tissue of the sub-topical layers"* as used in the present invention refers to the sub-topical layers of the skin excluding any blood vessels like capillaries and their vessel/capillary lumen.

Preferably, skin and/or the layer(s) of the skin have an expansion in the thickness direction and an expansion extending parallel to a surface of the skin and/or of the layer(s).

The expression *"expansion"* as used in the present invention refers to a linear, one-dimensional expansion of the skin and/or the layer(s) extending in parallel to the surface of the skin or in thickness direction of the skin.

An expansion parallel to the surface of the skin, as used in the present invention, is termed *"surface parallel expansion".* It may be, preferably, is expressed in cm (centimetre). It may refer to an expansion in the length or the width.

An expansion in thickness direction of the skin and/or of the layer(s) is, as used in the present invention, termed *"thickness".* The thickness may be, preferably, is expressed in cm (centimetre) and/or by stating one or more of the layers of the skin as defined above. The thickness of the skin and/or the layer(s) may vary in dependency of the genus and/or species a subject belongs to. Usually, in case the subject is a human, the skin has a thickness of 6 mm or less.

Preferably, in any one of the embodiments of the present invention, the skin is the skin of a skin area. Hence, more preferably,
step (A) refers to generating an accumulation of PBMCs, within a skin of a skin area; and
step (B) refers to administering the immunomodulatory substance, to the skin of the skin area; and
optional step (C) refers to administering the second immunomodulatory substance to the skin of the skin area.

The expression *"skin area"* as used in the present invention refers to a two-dimensional expansion extending in parallel to the surface of the skin. It may be, preferably, is indicated in cm² (square centimetres).

The skin area may have any, two-dimensional shape as long as the technical effect can be achieved and may have a certain localisation on the subject's body. For instance, the skin area may have a circular, triangular, rectangular, square, elliptic and/or polygonal shape, the shape may be, preferably, is irregular, lengthy and/or compact (the outline is rather short in respect of the encompassed skin area, e.g. a square a shorter outline than a rectangle in respect of the encompassed area)), it may have recesses and/or an outline with concave and/or convex parts or any combinations of these. Preferably, the skin area is approximately circular, square or rather compact in shape and more preferably does not have recesses.

The skin area may have any size as long as the technical effect can be achieved and may have even a size of 1.5 m² or less. Preferably, the skin area is 1000 cm² or less, more preferably 500 cm² or less, even more preferably 100 cm² or less, still more preferably 50 cm² or less, still even more preferably 25 cm² or less, still further preferably 10 cm², most preferably 5 cm² or less. Usually, the skin area is not smaller than 0.2 cm². Hence, a lower limit for the skin area is preferably 0.2 cm² or more, more preferably 0.5 cm² or more, even more preferably 1 cm² or more, still more preferably 2 cm² or more.

The expression *"skin of a*/*the skin area", "skin layer(s) of a*/*the skin area"* or the like as used in the present invention refers to the fragment of the skin or of one or more skin layers as defined above, covered by the skin area. For instance, the expression 'subcutis of the skin area' refers to the fragment of the subcutis covered by the skin area.

The expression *"on a*/*the skin"* as used in the present invention preferably refers to a topical treatment, administration, generation, application etc. performed on the surface of the skin, preferably the fragment skin which skin is covered by the skin area and/or the application area.

The expression *"topical"* refers to a treatment, administration, generation, application etc. performed on the surface of the skin.

The expressions *"into a*/*the skin"* and/or *"within a*/*the skin"* as used in the present invention preferably refers to a invasive treatment, administration, generation, application etc., for instance by injection, performed on the skin layers of the skin, preferably the fragment skin which skin is covered by the skin area and/or the application area.

The expression *"to a*/*the skin"* as used in the present invention preferably is the generic term covering the meaning of all three expressions *"on a*/*the skin", "into a*/*the skin"* and/or *"within a*/*the skin",* that is preferably refers to a topical and invasive treatment, administration, generation, application etc., for instance by injection, performed on the skin layers of the skin, preferably the fragment skin which skin is covered by the skin area and/or the application area.

Preferably, the skin and/or skin layer(s) of the skin area are at least partially, preferably, entirely immunological inactive and/or unchallenged and spatially distanced to any site of immunological activity and/or challenge.

Hence, the expression *"immunological inactive and*/*or unchallenged skin"* as used in the present invention refers skin and/or skin layer(s) of the skin area comprising, preferably consisting of a skin which is immunological inactivity and/or unchallenged and spatially distanced to any site of immunological activity and/or challenge

The expressions *"immunological inactive and*/*or unchallenged*" and/or *"absence of an immune challenge and*/*or activity"* as used in the present invention may mean, preferably, means that any kind of active immune response is absent and/or any stimulus or inducer of the immune system is present in an amount not sufficient to trigger an immune reaction or is absent, preferably an inflammation, an antigen, an autoantigen and/or an allergen is absent like for instance an inflamed joint, rheumatic joint, inflamed organ, allergic site, a wound and/or insect bite is absent, regardless whether it is caused immunologically or autoimmunologically or not. Hence, preferably, *"immunological inactive and*/*or unchallenged*" means that an inflammation, an antigen, an autoantigen and/or an allergen is absent.

The expression *"site of immunological activity and*/*or challenge"* as used in the present invention may be, preferably, is any site, where an active immune response is present and/or any stimulus or inducer of the immune system is present in an amount sufficient to trigger an immune reaction. Preferably, a site of immunological activity and/or challenge is a site where an inflammation, an antigen, an autoantigen and/or an allergen is present like for instance an inflamed joint, rheumatic joint, inflamed organ, allergic site, a wound and/or insect bite and it may be, preferably, is caused immunologically or autoimmunologically.

n any one of the embodiments of the present invention, more preferably, the spatial distance of the skin area to any site of immunological activity and/or challenge is 0.5 cm or more, more preferably 1 cm or more, even more preferably 3 cm or more, still more preferably, 5 cm or more and most preferably 10 cm or more. There is no upper limit for such distance, however, usually, it is not more than 1.5 m, more preferably 1 m. The distance is measures from a point on the outline of the skin area, which lies closest to a site of immunological activity and/or challenge. In case several sites of immunological activity and/or challenge are present, the antigen free skin area is preferably located spatially distanced to all these sites of immunological activity and/or challenge. Most preferably, the skin and/or skin layer(s) of the skin area are entirely immunological inactive and/or unchallenged and spatially distanced to any site of immunological activity and/or challenge as defined above.

The skin area or the skin and/or skin layer(s) of the skin area, which are entirely immunological inactive and/or unchallenged, may have any size as long as the technical effect can be achieved and may have a size of even about 1.5 m² or less. Preferably, the skin area is 1000 cm² or less, more preferably 500 cm² or less, even more preferably 100 cm² or less, still more preferably 50 cm² or less, still even more preferably 25 cm² or less, still further preferably 10 cm², most preferably 5 cm² or less. Usually, the skin area is not smaller than 0.5 cm². Hence, a lower limit for the skin area is preferably 0.5 cm² or more, more preferably 1 cm² or more, still more preferably 2 cm² or more.

In an aspect of all embodiments of the invention, step (A), step (B) and/or optional step (C), may be, preferably are accomplished by a topical administration and/or by injection as described in detail for each step further below. In case of an injection, the injection is preferably into the sub-topical layers, more preferably, into the dermis and/or subcutis, most preferably into the subcutis and/or the injection is preferably placed 6 mm or less to 1 mm or more, more preferably 5 mm or less to 1 mm or more, even more preferably 4 mm or less to 2 mm or more in skin thickness direction.

The expression *"application area"* as used in the present invention refers to the area in which the generating, administering, applying and/or injecting in step (A), step (B) and/or optional step (C) is accomplished and refers to a planer, two-dimensional expansion extending in parallel to the surface of the skin. In case of an injection, the application area may be, preferably, is the injection site. Preferably, the application area is congruent with the skin area and/or is arranged within the skin area, more preferably congruent. Hence, the application area may have a planar ring of skin area surrounding the application area. Hence, an outline of the application area may be spaced apart to an outline of the skin area, wherein the outline of the skin area lies outside the application area, or, in other word, the outline of the application area lies within the skin area, thereby the skin area is larger than the application area.

The skin area may be, preferably, is indicated in cm² (square centimetres). Reason for the planar ring is, that the substances and/or the effects of a physical treatment, like heat, applied to the skin within the application area may diffuse and/or radiate into the adjacent skin regions (planar ring) in a direction parallel to the skin surface and/or into the skin layer(s) (of course, a diffusion and/or radiation in skin thickness direction may also occur). Hence, the skin area in which skin the substances and/or effects of the physical treatment are still present in sufficient amounts and/or extent, may be larger (planar ring) than the actual application area. Preferably, the spacing between the outline of the application area and the skin area is 3 cm or less, more preferably 2 cm or less, even more preferably 1.5 cm or less, still more preferably 1 cm or less, still even more preferably 0.5 cm or less. Usually, there is no lower limit for the spacing and it may become even 0 cm in case where the application area is congruent with the skin area. It shall be pointed out that the spacing may or may not vary over the entire planar ring. Hence, the spacing is not necessarily constant for the entire ring, which may for instance depend on the constitution of the skin tissue surrounding the application area. Hence, the spacing may, preferably is, in a range of 0 cm to 3 cm, more preferably in 0.1 cm to 2 cm, even more preferably, 0.2 cm to 1.5 cm, still even more preferably, 0.3 cm to 1 cm. In case of an injection, the planar ring forms a disc around the point injection site, wherein the radius is the spacing.

The application area may have any two-dimensional shape as long as the technical effect can be achieved and may have a certain localisation on the subject's body. For instance, the application area may have a circular, triangular, rectangular, square, elliptic and/or polygonal shape, the shape may be, preferably, is irregular, lengthy and/or compact (the outline is rather short in respect of the encompassed application area, e.g. a square a shorter outline than a rectangle in respect of the encompassed area)), it may have recesses and/or an outline with concave and/or convex parts or any combinations of these. Preferably, the application area is approximately circular, square or rather compact in shape and more preferably does not have recesses.

The application area may have any size as long as the technical effect can be achieved and may have even a size of 1.5 m² or less. Preferably, the application area is 1000 cm² or less, more preferably 500 cm² or less, even more preferably 100 cm² or less, still more preferably 50 cm² or less, still even more preferably 25 cm² or less, still further preferably 10 cm², most preferably 5 cm² or less. Usually, the application area is not smaller than 0.2 cm². Hence, a lower limit for the application area is preferably 0.01 cm² or more (for instance in case of an injection), more preferably 0.02 cm² or more, even more preferably 1 cm² or more, still more preferably 2 cm² or more.

The expression *"skin of a*/*the application area", "skin layer(s) of a*/*the application area"* or the like as used in the present invention refers to the fragment of the skin or of one or more skin layers as defined above, covered by the application area. For instance, the expression 'subcutis of the application area' refers to the fragment of the subcutis covered by the application area.

Preferably, the skin and/or skin layer(s) of the application area are at least partially, preferably, entirely immunological inactive and/or unchallenged and spatially distanced to any site of immunological activity and/or challenge.

More preferably, the spatial distance of the application area to any site of immunological activity and/or challenge is 0.5 cm or more, more preferably 1 cm or more, even more preferably 3 cm or more, still more preferably, 5 cm or more and most preferably 10 cm or more. There is no upper limit for such distance, however, usually, it is not more than 1.5 m, more preferably 1 m. The distance is measures from a point on the outline of the application area, which lies closest to a site of immunological activity and/or challenge. In case several sites of immunological activity and/or challenge are present, the antigen free application area is preferably located spatially distanced to all these sites of immunological activity and/or challenge. Most preferably, the skin and/or skin layer(s) of the application area are entirely immunological inactive and/or unchallenged and spatially distanced to any site of immunological activity and/or challenge as defined above.

The application area or the skin and/or skin layer(s) of the application area, which are entirely immunological inactive and/or unchallenged, may have any size as long as the technical effect can be achieved and may have a size of even about 1.5 m² or less. Preferably, the application area is 1000 cm² or less, more preferably 500 cm² or less, even more preferably 100 cm² or less, still more preferably 50 cm² or less, still even more preferably 25 cm² or less, still further preferably 10 cm², most preferably 5 cm² or less. Usually, the application area is not smaller than 0.5 cm². Hence, a lower limit for the application area is preferably 0.5 cm² or more, more preferably 1 cm² or more, still more preferably 2 cm² or more.

More preferably, the skin is in step (A) a skin of a skin area [a], the skin is in step (B) a skin of a skin area [b] and/or the skin is in step (C) a skin of a skin area [c]. Still more preferably,
step (A) refers to generating an accumulation of PBMCs within a skin of a skin area by generating the accumulation within a skin of a skin area [a]; and
step (B) refers to administering the immunomodulatory substance to the skin of the skin area by administering to a skin of a skin area [b]; and
optional step (C) refers to administering the second immunomodulatory substance to the skin of the skin area by administering to a skin of a skin area [c].

The skin areas [a], [b] and/or [c] are defined as described below.

Preferably, the skin area [a] and the skin area [b] overlap and optional the skin area [a], the skin area [b] and the skin area [c] overlap.

Preferably, the method/the method of the medical use is performed within 5 h or less, more preferably within 3 h or less, even more preferably within 1 h or less, still more preferably within 0.5 h or less, even further preferred within 15 min or less, still even further preferably within 5 min or less, most preferably, within 1 min or less.

Preferably, the method/the method of the medical use is performed at a frequency of once per day or less often, more preferably once per 2 days or less often, even more preferably once per 3 days or less often, still more preferably once per 4 days or less often, still even more preferably once per 5 days or less often, furthermore preferably once per week or less often, still furthermore preferably once per 2 weeks or less often, most preferably once per month or less often. Usually, there is no upper limit for the frequency as long the technical effect can be achieved, however, an upper limit may be, preferably, is that method/the method of the medical use is performed at a frequency of once per two months or more often, more preferably, once per 6 weeks or more often. Preferably, the method/the method of the medical use is performed at a frequency of once per day to one per two months, more preferably once per 3 day to once per 6 weeks, even more preferably once per 5 days to once per month.

### Regarding step (A)

Preferably, the accumulation is generated within the lumen of the capillaries of the skin.

More preferably, the accumulation is generated within the sub-topical layers, even more preferably the dermis and/or subcutis of the skin, still more preferably, within the lumen of the capillaries of the sub-topical layers, mist preferably the dermis and/or subcutis.

As stated above, PBMCs, in particular lymphocytes and/or naïve T-cells are not present or at least not in effective amounts within the skin capillaries and hence within the skin. Hence, the presence of PBMCs, preferably lymphocytes, more preferably naïve T-cells within the skin may, preferably, is already regarded as accumulation of PBMCs, preferably lymphocytes, more preferably naïve T-cells in step (A).

The accumulation of PBMCs, lymphocytes, T-cells, and naïve T-cells can be measured by any method known to the person skilled in the art suitable to count and/or otherwise quantify such cells within the skin.

Preferably, in step (A) the accumulation of PBMCs, is generated within the sub-topical layers of the skin, more preferably within the dermis and/or the subcutis, still more preferably within the subcutis.

Preferably, in the step (A) the generating of the accumulation of PBMCs may be, preferably, is accomplished by any means, method, substance(s) and/or procedure, wherein the means, method, substance(s) and/or procedure is not particularly limited as long as it is suitable to generate an accumulation of PBMCs, preferably lymphocytes, within the skin.

Preferably, in step (A) the accumulation of PBMCs, preferably, lymphocytes is generated by:
- (A-1): generating a vasodilation of the capillaries within the skin; and/or
- (A-2): generating an increased blood flow within the skin, wherein the blood flow is increased by 1% or more, preferably when measured as described in the methods, item 3 'measurement of the blood flow' and/or is increased by 10 µl/s or more, preferably when measured as described in the methods, item 3 'measurement of the blood flow' and/or
- (A-3): generating an increased temperature on the skin, wherein preferably the temperature is increased by 1 % or more and/or is increased by 0.2 °C or more, preferably when measured as described in the methods 'measurement of the temperature of the skin'; and/or
- (A-4): generating a redness on the skin, wherein the redness is visually detectable to the naked eye; and/or
- (A-5): applying heat to the skin; and/or
- (A-6): applying mechanical agitation to the skin; and/or
- (A-7): applying negative pressure to the skin; and/or
- (A-8): administering a blood-circulation-increasing agent to the skin; and/or
- (A-9): administering a vasodilating agent to the skin; and/or
- (A-10): injecting PBMCs into the skin, preferably the skin of the skin area.

Preferably, in step (A), and hence in and steps (A-1) to (A-10), the skin are is a skin area [a] and, if applicable, the application area is an application area [a].

Preferably, the skin area [b] and the application area [b] are *mutatis mutandis* defined as the skin area [a] and the application area [a], respectively.

### As regards step (A-1)

As stated above, (A-1) requires generating a vasodilation of the capillaries within the skin.

Preferably, in step (A-1), the generating of a vasodilation of the capillaries may be, preferably is accomplished by any means, method, substance(s) and/or procedure as long as it is suitable to generate a vasodilation of the capillaries within the skin and without damaging the skin like rupture of capillaries and/or reflexive closure of the capillaries.

The vasodilation may be to any extent as long as PBMCs can enter the capillaries. The presence of PBMCs, more preferably lymphocytes within the capillaries of the skin can be measured by any method known to the person skilled in the art suitable to count and/or otherwise quantify such cells within the skin.

Preferably, the vasodilation is of the capillaries in the sub-topical layers, more preferably the dermis and/or subcutis, more preferably within the subcutis.

Preferably, in step (A-1) the vasodilation is indicated by:
- an increased blood flow within the skin, preferably, the dermis, by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2); and/or
- an increased temperature on the skin, wherein preferably the temperature is increased by 1 % or more and/or is increased by 0.2 °C or more, preferably when measured as described in the methods 'measurement of the temperature of the skin', wherein both are preferably *mutatis mutandis* accomplished and/or defined as described under the topic 'As regards step (A-3); and/or
- a redness on the skin, wherein the redness is visually detectable to the naked eye, preferably *mutatis mutandis* accomplished and/or defined as described under the topic 'As regards step (A-4).

Preferably, in step (A-1) the generating of the vasodilation of the capillaries is effected by:
- generating an increased blood flow within the skin, preferably, the dermis, by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2); and/or
- generating an increased temperature on the skin, wherein preferably the temperature is increased by 1 % or more and/or is increased by 0.2 °C or more, wherein both are preferably *mutatis mutandis* accomplished and/or defined as described under the topic 'As regards step (A-3); and/or
- generating a redness on the skin, wherein the redness is visually detectable to the naked eye, preferably *mutatis mutandis* accomplished and/or defined as described under the topic 'As regards step (A-4); and/or
- applying heat to the skin, preferably *mutatis mutandis* accomplished and/or defined as described under the topic 'As regards step (A-5); and/or
- applying mechanical agitation to the skin (to the skin surface), preferably *mutatis mutandis* accomplished and/or defined as described under the topic 'As regards step (A-6); and/or
- applying negative pressure to the skin, preferably *mutatis mutandis* accomplished and/or defined as described under the topic 'As regards step (A-7); and/or
- administering a blood-circulation-increasing agent to the skin, preferably *mutatis mutandis* accomplished and/or defined as described under the topic 'As regards step (A-8); and/or
- administering a vasodilating agent to the skin, preferably *mutatis mutandis* accomplished and/or defined as described under the topic 'As regards step (A-9).

For the indication of the vasodilation and/or the effecting of the vasodilation, the blood flow within the skin is increased:
- more preferably by 2 %, even more preferably by 5 %, still even more preferably by 10 %, preferably when measured as described in the methods, item 3 'measurement of the blood flow', wherein there is no upper limit for the increase in blood flow as long as no burning lesions and/or damaging of skin is caused, however, usually, the increase of the blood flow is 50-fold or less, preferably 30-fold or less; and/or
- more preferably by 50 ml/min/100 g, even more preferably by 100 ml/min/100 g, still even more preferably by 200 ml/min/100 g, preferably when measured as described in the methods, item 3 'measurement of the blood flow', wherein there is no upper limit for the increase in blood flow as long as no burning lesions and/or damaging of skin is caused, however, usually, the increase of the blood flow is 800 ml/min/100 g or less, preferably 400 ml/min/100 g or less.

Preferably, the increase of the blood flow refers to the blood flow of untreated skin, preferably of an arm or leg, more preferably of a human.

Furthermore, for the indication of the vasodilation and/or the effecting of the vasodilation, the temperature on the skin is increased:
- more preferably by 3 % or more, even more preferably by 5 % or more, still more preferably by 8 % or more, most preferably by 10 % or more, most preferably by 13 % or more, when referred to °C (degrees Celsius) and preferably measured as described in the methods 'measurement of the temperature of the skin', wherein there is no upper limit for the increase as long as no burning lesions and/or damaging of skin is caused, however, usually, the increase of the temperature is 20 % or less, preferably 15 % or less; and/or
- more preferably by 0.3 °C or more, even more preferably by 0.5 °C or more, still more preferably by 0.8 °C or more, furthermore preferably by 1 °C or more, still furthermore preferably by 1.5 °C or more, most preferably by 3 °C or more, preferably, preferably when measured as described in the methods 'measurement of the temperature of the skin', wherein there is no upper limit for the increase as long as no burning lesions and/or damaging of skin is caused, however, usually, the increase of the temperature is 20 °C or less, preferably 10 °C or less, even more preferably 8 °C or less, still more preferably 5 °C or less.

Preferably, the increase of the temperature on the skin refers to the temperature of untreated skin, preferably of an arm or leg, more preferably of a human. Preferably, untreated skin has a temperature of 34 °C or less, more preferably in the range of 20 °C to 34 °C, even more preferably 28 °C to 34°C.

### As regards step (A-2)

As stated above, (A-2) requires generating an increased blood flow within the skin, wherein preferably the blood flow increased blood flow within the skin, preferably, the dermis, by 1 % or and/or by more10 ml/min/100 g, preferably when measured as described in the methods, item 3 'measurement of the blood flow'.

Preferably, the blood flow is increased within the capillaries.

Preferably, the blood flow is increased in the sub-topical layers, more preferably the dermis and/or subcutis, more preferably within the subcutis.

More preferably, the blood flow within the skin is increased: :
- more preferably by 2 %, even more preferably by 5 %, still even more preferably by 10 %, preferably when measured as described in the methods, item 3 'measurement of the blood flow', wherein there is no upper limit for the increase in blood flow as long as no burning lesions and/or damaging of skin is caused, however, usually, the increase of the blood flow is 50-fold or less, preferably 30-fold or less; and/or
- more preferably by 50 ml/min/100 g, even more preferably by 100 ml/min/100 g, still even more preferably by 200 ml/min/100 g, preferably when measured as described in the methods, item 3 'measurement of the blood flow', wherein there is no upper limit for the increase in blood flow as long as no burning lesions and/or damaging of skin is caused, however, usually, the increase of the blood flow is 800 ml/min/100 g or less, preferably 400 ml/min/100 g or less.

Preferably, the increase of the blood flow refers to the blood flow of untreated skin, preferably of an arm or leg, more preferably of a human.

Preferably, in step (A-2) the increased blood flow is indicated by:
- a vasodilation of the capillaries within the skin, preferably *mutatis mutandis* accomplished and/or defined as described under the topic 'As regards step (A-1); and/or
- an increased temperature on the skin, wherein preferably the temperature is increased by 1 % or more and/or is increased by 0.2 °C or more, preferably when measured as described in the methods 'measurement of the temperature of the skin', wherein both are preferably *mutatis mutandis* accomplished and/or defined as described under the topic 'As regards step (A-3); and/or
- a redness on the skin, wherein the redness is visually detectable to the naked eye, preferably *mutatis mutandis* accomplished and/or defined as described under the topic 'As regards step (A-4).

Preferably, in step (A-2) the generating of the increased blood flow is effected by:
- generating a vasodilation of the capillaries within the skin, preferably *mutatis mutandis* accomplished and/or defined as described under the topic 'As regards step (A-1); and/or
- generating an increased temperature on the skin, wherein preferably the temperature is increased by 1 % or more and/or is increased by 0.2 °C or more, preferably when measured as described in the methods 'measurement of the temperature of the skin', wherein both are preferably *mutatis mutandis* accomplished and/or defined as described under the topic 'As regards step (A-3); and/or
- generating a redness on the skin, wherein the redness is visually detectable to the naked eye, preferably *mutatis mutandis* accomplished and/or defined as described under the topic 'As regards step (A-4); and/or
- applying heat to the skin, preferably *mutatis mutandis* accomplished and/or defined as described under the topic 'As regards step (A-5); and/or
- applying mechanical agitation to the skin, preferably *mutatis mutandis* accomplished and/or defined as described under the topic 'As regards step (A-6); and/or
- applying negative pressure to the skin, preferably *mutatis mutandis* accomplished and/or defined as described under the topic 'As regards step (A-7); and/or
- administering a blood-circulation-increasing agent to the skin, preferably *mutatis mutandis* accomplished and/or defined as described under the topic 'As regards step (A-8); and/or
- administering a vasodilating agent to the skin, preferably *mutatis mutandis* accomplished and/or defined as described under the topic 'As regards step (A-9).

### As regards step (A-3)

As stated above, (A-3) requires generating an increased temperature on the skin, wherein preferably the temperature is increased by 1 % or more and/or is increased by 0.2 °C or more, preferably when measured as described in the methods 'measurement of the temperature of the skin'.

Preferably, in step (A-3) the increased temperature on the skin is indicated by:
- a vasodilation of the capillaries within the skin, preferably *mutatis mutandis* accomplished and/or defined as described under the topic 'As regards step (A-1); and/or
- an increased blood flow within the skin, preferably, the dermis, by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2);; and/or
- a redness on the skin, wherein the redness is visually detectable to the naked eye, preferably *mutatis mutandis* accomplished and/or defined as described under the topic 'As regards step (A-4).

Preferably, in step (A-3) the generating of an increased temperature is effected by:
- generating a vasodilation of the capillaries within the skin, preferably *mutatis mutandis* accomplished and/or defined as described under the topic 'As regards step (A-1); and/or
- generating an increased blood flow within the skin, preferably, the dermis, by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2);; and/or
- generating a redness on the skin, wherein the redness is visually detectable to the naked eye, preferably *mutatis mutandis* accomplished and/or defined as described under the topic 'As regards step (A-4); and/or
- applying heat to the skin, preferably *mutatis mutandis* accomplished and/or defined as described under the topic 'As regards step (A-5); and/or
- applying mechanical agitation to the skin, preferably *mutatis mutandis* accomplished and/or defined as described under the topic 'As regards step (A-6); and/or
- applying negative pressure to the skin, preferably *mutatis mutandis* accomplished and/or defined as described under the topic 'As regards step (A-7); and/or
- administering a blood-circulation-increasing agent to the skin, preferably *mutatis mutandis* accomplished and/or defined as described under the topic 'As regards step (A-8); and/or
- administering a vasodilating agent to the skin, preferably *mutatis mutandis* accomplished and/or defined as described under the topic 'As regards step (A-9).

### As regards step (A-4)

As stated above, (A-4) requires generating a redness on the skin.

Preferably, the redness is visually detectable to the naked eye.

Preferably, the redness is on the surface of the skin.

Preferably, the redness is visually detectable to the naked eye under day light conditions and/or artificial light conditions.

Preferably, in step (A-3) the redness on the skin is indicated by:
- a vasodilation of the capillaries within the skin, preferably *mutatis mutandis* accomplished and/or defined as described under the topic 'As regards step (A-1); and/or
- an increased blood flow within the skin, preferably, the dermis, by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2);; and/or
- an increased temperature on the skin, wherein preferably the temperature is increased by 1 % or more and/or is increased by 0.2 °C or more, preferably when measured as described in the methods 'measurement of the temperature of the skin', preferably *mutatis mutandis* accomplished and/or defined as described under the topic 'As regards step (A-3).

Preferably, in step (A-4) the generating of the redness is effected by:
- generating a vasodilation of the capillaries within the skin, preferably *mutatis mutandis* accomplished and/or defined as described under the topic 'As regards step (A-1); and/or
- generating an increased blood flow within the skin, preferably, the dermis, by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2); and/or
- generating an increased temperature on the skin, wherein preferably the temperature is increased by 1 % or more and/or is increased by 0.2 °C or more, preferably when measured as described in the methods 'measurement of the temperature of the skin', wherein both are preferably *mutatis mutandis* accomplished and/or defined as described under the topic 'As regards step (A-3); and/or
- applying heat to the skin, preferably *mutatis mutandis* accomplished and/or defined as described under the topic 'As regards step (A-5); and/or
- applying mechanical agitation to the skin, preferably *mutatis mutandis* accomplished and/or defined as described under the topic 'As regards step (A-6); and/or
- applying negative pressure to the skin, preferably *mutatis mutandis* accomplished and/or defined as described under the topic 'As regards step (A-7); and/or
- administering a blood-circulation-increasing agent to the skin, preferably *mutatis mutandis* accomplished and/or defined as described under the topic 'As regards step (A-8); and/or
- administering a vasodilating agent to the skin, preferably *mutatis mutandis* accomplished and/or defined as described under the topic 'As regards step (A-9).

### As regards step (A-5)

As stated above, (A-5) requires applying heat to the skin, preferably to the surface of the skin.

Preferably, the heat is applied by any means known to the person skilled in the art which do not cause lesions and/or a damaging of skin. For instance, the heat is applied by using heating means like radiation like infrared radiation, moxibutation, hyperthermia, manual skin massage using vibration and/or rubbing of the skin, a warm object like a heater, a heat pad or a preheated object, a hot air, warm tea cup, etc.

Preferably, the heat is applied to an extent and/or a duration suitable to achieve the technical effect, is pharmaceutically acceptable and does not cause lesions and/or a damaging of skin like rupture of capillaries and/or reflexive closure of the capillaries.

Preferably, the heat has a temperature of 30 °C or more, more preferably of 34 °C or more, even more preferably of 36 °C or more, still more preferably of 38 °C or more, most preferably of 40 °C or more. There is no upper limit for the temperature as long as no burning lesions and/or damaging of skin is caused however, usually the temperature is 65 °C or less, preferably 58 °C or less, more preferably 45 °C or less, still more preferably 42 °C or less, most preferably 41 °C or less.

Preferably, the heating means have a temperature and/or are suitable to create a temperature on the skin of 30°C or more, more preferably of 34 °C or more, even more preferably of 36 °C or more, still more preferably of 38 °C or more, most preferably of 40 °C or more. There is no upper limit for the temperature as long as no burning lesions and/or damaging of skin is caused however, usually the temperature is 65 °C or less, preferably 58 °C or less, more preferably 45 °C or less, still more preferably 42 °C or less, most preferably 41 °C or less. Preferably, the temperature on the skin can, more preferably is, measured as described in the methods 'measurement of the temperature of the skin'.

Preferably, the heat is applied for a duration of 60 min or less, more preferably of 30 min or less, even more preferably of 15 min or less, still more preferably of 5 min or less. Usually, it is applied for a duration of 30 sec or more, more preferably of 1 min or more.

Preferably, the heat is applied to an extent and/or a duration sufficient to generate a vasodilation of the capillaries within the skin, preferably the sub-topical layers of the skin, more preferably the dermis and subcutis, most preferably the subcutis, wherein the vasodilation of the capillaries can be measured by any method known to the person skilled in the art.

Preferably, the heat is applied to an extent and/or a duration sufficient to generate:
- a vasodilation of the capillaries within the skin, preferably *mutatis mutandis* accomplished and/or defined as described under the topic 'As regards step (A-1); and/or
- an increased blood flow within the skin, preferably, the dermis, by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2); and/or
- an increased temperature on the skin, wherein preferably the temperature is increased by 1 % or more and/or is increased by 0.2 °C or more, preferably when measured as described in the methods 'measurement of the temperature of the skin', preferably *mutatis mutandis* accomplished and/or defined as described under the topic 'As regards step (A-3); and/or
- a redness on the skin, wherein the redness is visually detectable to the naked eye, preferably *mutatis mutandis* accomplished and/or defined as described under the topic 'As regards step (A-4).

Preferably, the applying of the heat to the skin may be, preferably, is accomplished by a topical administration.

### As regards step (A-6)

As stated above, (A-6) requires applying mechanical agitation to the skin, preferably to the surface of the skin.

Preferably, the mechanical agitation is applied by any means known to the person skilled in the art which do not cause lesions and/or a damaging of skin like rupture of capillaries and/or reflexive closure of the capillaries. For instance, the mechanical agitation is applied by, manual skin massage using vibration and/or rubbing of the skin, machines suitable for skin massage like a facial massager, vibrating machines etc.

Preferably, the mechanical agitation is applied to an extent and/or a duration suitable to achieve the technical effect, is pharmaceutically acceptable and does not cause lesions and/or a damaging of skin like rupture of capillaries and/or reflexive closure of the capillaries.

Preferably, the mechanical agitation may be continuous and/or pulsed.

Preferably, the mechanical agitation is applied for a duration of 60 min or less, more preferably of 30 min or less, even more preferably of 15 min or less, still more preferably of 5 min or less. Usually, it is applied for a duration of 30 sec or more, more preferably of 1 min or more.

Preferably, the mechanical agitation is applied to an extent and/or a duration sufficient to generate a vasodilation of the capillaries within the skin, preferably the sub-topical layers of the skin, more preferably the dermis and subcutis, most preferably the subcutis, wherein the vasodilation of the capillaries can be measured by any method known to the person skilled in the art.

Preferably, the mechanical agitation is applied to an extent and/or a duration sufficient to generate an increased blood flow within the skin, preferably the sub-topical layers of the skin, more preferably the dermis and subcutis, most preferably the subcutis, wherein preferably the blood flow is increased by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2).

Preferably, the mechanical agitation is applied to an extent and/or a duration sufficient to generate:
- a vasodilation of the capillaries within the skin, preferably *mutatis mutandis* accomplished and/or defined as described under the topic 'As regards step (A-1); and/or
- generating an increased blood flow within the skin, preferably, the dermis, by 1% or and/or by more 10 ml/min/100 g, preferably when measured as described in the methods, item 3 'measurement of the blood flow', wherein both are preferably *mutatis mutandis* accomplished and/or defined as described under the topic 'As regards step (A-2);; and/or
- an increased temperature on the skin, wherein preferably the temperature is increased by 1 % or more and/or is increased by 0.2 °C or more, preferably when measured as described in the methods 'measurement of the temperature of the skin', preferably *mutatis mutandis* accomplished and/or defined as described under the topic 'As regards step (A-3); and/or
- a redness on the skin, wherein the redness is visually detectable to the naked eye, preferably *mutatis mutandis* accomplished and/or defined as described under the topic 'As regards step (A-4).

Preferably, the applying of the mechanical agitation to the skin may be, preferably, is accomplished by a topical application.

### As regards step (A-7)

As stated above, (A-7) requires applying negative pressure to the skin (to the skin surface, preferably to the surface of the skin.

Preferably, the negative pressure is applied by any means known to the person skilled in the art which are suitable to create a vacuum on the skin and which do not cause lesions and/or a damaging of skin like rupture of capillaries and/or reflexive closure of the capillaries. For instance, the negative pressure is applied by sucking, cupping using for instance cupping glasses or a cupping machine and/or by applying any kind of vacuum, preferably cupping, more preferably dry cupping and/or cupping without injuring the skin.

Preferably, the negative pressure is applied to an extent and/or a duration suitable to achieve the technical effect, is pharmaceutically acceptable and does not cause lesions and/or a damaging of skin like rupture of capillaries and/or reflexive closure of the capillaries.

Preferably, the negative pressure is in an extent in the range of 0 hPa to 1000 hPa, more preferably 0 hPa to 600 hPa.

Preferably, the negative pressure may be continuous and/or pulsed.

Preferably, the negative pressure is applied for a duration of 60 min or less, more preferably of 30 min or less, even more preferably of 15 min or less, still more preferably of 5 min or less. Usually, it is applied for a duration of 30 sec or more, more preferably of 1 min or more.

Preferably, the negative pressure is applied to an extent and/or a duration sufficient to generate a vasodilation of the capillaries within the skin, preferably the sub-topical layers of the skin, more preferably the dermis and subcutis, most preferably the subcutis, wherein the vasodilation of the capillaries can be measured by any method known to the person skilled in the art.

Preferably, the negative pressure is applied to an extent and/or a duration sufficient to generate an increased blood flow within the skin, preferably the sub-topical layers of the skin, more preferably the dermis and subcutis, most preferably the subcutis, wherein preferably the blood flow is increased 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2);.

Preferably, the negative pressure is applied to an extent and/or a duration sufficient to generate:
- a vasodilation of the capillaries within the skin, preferably *mutatis mutandis* accomplished and/or defined as described under the topic 'As regards step (A-1); and/or
- an increased blood flow within the skin, preferably, the dermis, by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2); and/or
- an increased temperature on the skin, wherein preferably the temperature is increased by 1 % or more and/or is increased by 0.2 °C or more, preferably when measured as described in the methods 'measurement of the temperature of the skin', preferably *mutatis mutandis* accomplished and/or defined as described under the topic 'As regards step (A-3); and/or
- a redness on the skin, wherein the redness is visually detectable to the naked eye, preferably *mutatis mutandis* accomplished and/or defined as described under the topic 'As regards step (A-4).

Preferably, the applying of the negative pressure to the skin may be, preferably, is accomplished by a topical application.

### As regards step (A-8)

As stated above, (A-8) requires administering a blood-circulation-increasing agent to the skin.

Preferably, the blood-circulation-increasing agent is any substance, composition or formulation suitable to dilate the vessels and capillaries, particularly the capillaries, of the skin. Examples for blood-circulation-increasing agents comprise nitrates, alpha blockers, ACE-inhibitors, ginkgo preparations like ginko balm, calcium antagonists, dihydralazine, minoxidil, dihydroergotoxin, nicotinic acid analogues, methylnicotinat, capsaicine, and/or pentoxifylline, Kytta heat balm (by P&G Health Germany GmbH), and/or combinations thereof. Preferably, the vasodilating agent is Kytta heat balm and/or methylnicotinat.

Preferably, the blood-circulation-increasing agent is administered in an amount and/or for a duration suitable to achieve the technical effect, which is pharmaceutically acceptable and does not cause lesions and/or a damaging of skin.

Preferably, the negative pressure is applied to an extent and/or a duration suitable to achieve the technical effect, is pharmaceutically acceptable and does not cause lesions and/or a damaging of skin like rupture of capillaries and/or reflexive closure of the capillaries.

More preferably, the blood-circulation-increasing agent is applied for a duration of 60 min or less, more preferably of 30 min or less, even more preferably of 15 min or less, still more preferably of 5 min or less. Usually, it is applied for a duration of 30 sec or more, more preferably of 1 min or more.

Preferably, the blood-circulation-increasing agent is administered in an amount and/or for a duration sufficient to generate a vasodilation of the capillaries within the skin, preferably the sub-topical layers of the skin, more preferably the dermis and subcutis, wherein the vasodilation of the capillaries can be measured by any method known to the person skilled in the art.

Preferably, the blood-circulation-increasing agent is administered in an amount and/or for a duration sufficient to generate an increased blood flow within the skin, preferably the sub-topical layers of the skin, more preferably the dermis and subcutis, wherein preferably the blood flow increased by 1% or and/or by more 10 ml/min/100 g, preferably when measured as described in the methods, item 3 'measurement of the blood flow', wherein both are preferably *mutatis mutandis* accomplished and/or defined as described under the topic 'As regards step (A-2);.

Preferably, the blood-circulation-increasing agent is administered in an amount and/or for a duration sufficient to generate:
- a vasodilation of the capillaries within the skin, preferably *mutatis mutandis* accomplished and/or defined as described under the topic 'As regards step (A-1); and/or
- an increased blood flow within the skin, preferably, the dermis, by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2); and/or
- an increased temperature on the skin, wherein preferably the temperature is increased by 1 % or more and/or is increased by 0.2 °C or more, preferably when measured as described in the methods 'measurement of the temperature of the skin', preferably *mutatis mutandis* accomplished and/or defined as described under the topic 'As regards step (A-3); and/or
- a redness on the skin, wherein the redness is visually detectable to the naked eye, preferably *mutatis mutandis* accomplished and/or defined as described under the topic 'As regards step (A-4).

Preferably, the administrating of the blood-circulation-increasing agent to the skin may be, preferably, is accomplished by a topical administration and/or by injection, more preferably by a topical administration.

In case of an injection, the injection is preferably into the sub-topical layers, more preferably, into the dermis and subcutis, most preferably into the subcutis and/or the injection is preferably placed 6 mm or less to 1 mm or more, more preferably 5 mm or less to 1 mm or more, even more preferably 4 mm or less to 2 mm or more in skin thickness direction. Preferably, the injection is placed into the upper arm, forearm and/or upper leg.

Preferably, for injection, a composition as described herein is used.

Preferably, for the topical administration, a composition as described herein is used.

### As regards step (A-9)

As stated above, (A-9) requires administering a vasodilating agent to the skin.

Preferably, the vasodilating agent is any substance, composition or formulation suitable to dilate the vessels and capillaries, particularly the capillaries, of the skin. Examples for vasodilating agents comprise nitrates, alpha blockers, ACE-inhibitors, ginkgo preparations like ginko balm, calcium antagonists, dihydralazine, minoxidil, dihydroergotoxin, nicotinic acid analogues, methylnicotinat, capsaicine, and/or pentoxifylline, Kytta heat balm (by P&G Health Germany GmbH), and/or combinations thereof. Preferably, the vasodilating agent is Kytta heat balm and/or methylnicotinat.

Preferably, the vasodilating agent is administered in an amount and/or for a duration suitable to achieve the technical effect, is pharmaceutically acceptable and does not cause lesions and/or a damaging of skin. More preferably, the vasodilating agent is methylnicotinat.

More preferably, the vasodilating agent is applied for a duration of 60 min or less, more preferably of 30 min or less, even more preferably of 15 min or less, still more preferably of 5 min or less. Usually, it is applied for a duration of 30 sec or more, more preferably of 1 min or more.

Preferably, the vasodilating agent is administered in an amount and/or for a duration sufficient to generate a vasodilation of the capillaries within the skin, preferably the sub-topical layers of the skin, more preferably the dermis and subcutis.

Preferably, the vasodilating agent is administered in an amount and/or for a duration sufficient to generate an increased blood flow within the skin, preferably the sub-topical layers of the skin, more preferably the dermis and subcutis, wherein preferably the blood flow is increased by 1% or and/or by more10 ml/min/100 g, preferably when measured as described in the methods 'measurement of the vasodilation', the capillaries can be measured by any method known to the person skilled in the art.

Preferably, the vasodilating agent is administered in an amount and/or for a duration sufficient to generate:
- a vasodilation of the capillaries within the skin, preferably *mutatis mutandis* accomplished and/or defined as described under the topic 'As regards step (A-1); and/or
- an increased blood flow within the skin, preferably, the dermis, by 1% or and/or by more10 ml/min/100 g, preferably when measured as described in the methods, item 3 'measurement of the blood flow', wherein both are preferably *mutatis mutandis* accomplished and/or defined as described under the topic 'As regards step (A-2); and/or
- an increased temperature on the skin, wherein preferably the temperature is increased by 1 % or more and/or is increased by 0.2 °C or more, preferably when measured as described in the methods 'measurement of the temperature of the skin', preferably *mutatis mutandis* accomplished and/or defined as described under the topic 'As regards step (A-3); and/or
- a redness on the skin, wherein the redness is visually detectable to the naked eye, preferably *mutatis mutandis* accomplished and/or defined as described under the topic 'As regards step (A-4).

Preferably, the administrating of the vasodilating agent to the skin may be, preferably, is accomplished by a topical administration and/or by injection, more preferably by a topical administration.

In case of a topical administration, this is preferably placed on the upper arm, forearm and/or upper leg.

In case of an injection, the injection is preferably into the sub-topical layers, more preferably, into the dermis and subcutis, most preferably into the subcutis and/or the injection is preferably placed 6 mm or less to 1 mm or more, more preferably 5 mm or less to 1 mm or more, even more preferably 4 mm or less to 2 mm or more in skin thickness direction. Preferably, the injection is placed into the upper arm, forearm and/or upper leg.

Preferably, for injection, a composition as described herein is used.

Preferably, for the topical administration, a composition as described herein is used.

### As regards step (A-10)

As stated above, (A-10) requires injecting PBMCs into the skin.

Preferably, the PBMCs are defined an obtained as described above. More preferably, the PBMCs are blood PBMCs or isolated PBMCs, even more preferably isolated PBMCs. Still more preferably, the PBMCs are lymphocytes, still even more preferably, naïve lymphocytes, most preferably naïve T-cells. PBMCs may be, preferably, are prepared as described in the methods 'Preparation of PBMCs'.

The PBMCs may be formulated in any kind of formulation which is pharmaceutical acceptable and suitable for injection, preferably injection into the subject, as long as the PBMCs are maintained alive or at least an effective amount of PBMCs are maintained alive. Preferably, the PBMCs may be, preferably, are formulated as described in the methods 'Preparation of PBMCs'.

The PBMCs may by freshly prepared or they may have been frozen, preferably in liquid nitrogen, and thawed and reconstituted prior to use in media and/or solutions in order to obtain the formulation. Preferably, the PBMCs may be, preferably, are formulated as described in the methods 'Preparation of PBMCs'.

Preferably, the PBMCs have a concentration in the formulation of 50×10⁶ PBMCs/ml or less, more preferably of 20×10⁶ PBMCs/ml or less.

Preferably, the PBMCs are injected in a amount of 1×10⁵ PBMCs or more, more preferably 1×10⁶ PBMCs or more, even more preferably 2×10⁶ PBMCs or more, still more preferably 4×10⁶ PBMCs or more per injection. There is no upper limit for the amount of PBMCs, however, usually, an upper limit may be, preferably, is 1×10⁷ PBMCs or less per injection.

Preferably, the PBMCs are injected in a total amount of 2×10⁵ PBMCs or more, more preferably 2×10⁶ PBMCs or more, even more preferably 4×10⁶ PBMCs or more, still more preferably 8×10⁶ PBMCs or more. There is no upper limit for the amount of PBMCs, however, usually, an upper limit may be, preferably, is 2×10⁷ PBMCs or less.

Preferably, the PBMCs are injected into the sub-topical layers of the skin, more preferably, into the dermis and subcutis, most preferably into the subcutis of the skin and/or the injection is preferably placed 6 mm or less to 1 mm or more, more preferably 5 mm or less to 1 mm or more, even more preferably 4 mm or less to 2 mm or more in skin thickness direction. Preferably, the injection is placed into the upper arm, forearm and/or upper leg.

### Regarding step (B)

As already stated above, the method/the method of the medical use according to the present invention preferably comprises the further step of:
(B) administering an immunomodulatory substance, preferably IFN-γ to the skin.

Preferably, IFN-γ is administered by administering the IFN-γ or a composition comprising IFN-γ.

Generally, 1 ng IFN-γ corresponds to 20 IU IFN-γ or less. Some of the IFN-γ-protein might be denatured or otherwise defect in that the biological activity of IFN-γ has been lost while the mass remains constant.

It is intended to administer IFN-γ in an amount low enough to avoid a systemic increase of the IFN-γ-concentration in the subject's body. Thereby, according to theory, it is avoided to generate an increased IFN-γ-concentration as for instance at sites of inflammations, like inflamed joint. As already stated above, for instance naïve T-cells mature in the presence of antigen/autoantigen/allergen towards cytotoxic or helper T-cells, which may act proinflammatory thereby decreasing, cancelling or even reversing the beneficial effects achieved by the method of the medical use according to the present invention, particularly, the anti-inflammatory effect of the regulatory T-cells presumably generated.

Preferably, IFN-γ is administered in an amount of 600 IU/kg body mass of a subject or less per administration dose to a skin of the subject. More preferably, IFN-γ is administered in an amount of 300 IU/kg or less, even more preferably of 200 IU/kg or less, still more preferably of 100 IU/kg or less, still even more preferably 50 IU/kg or less, furthermore preferably 30 IU/kg or less, most preferably 20 IU/kg or less. There is no lower limit for the amount per kg body mass as long as the technical effect can be achieved, however, usually, a lower limit is 0.5 IU/kg or more, more preferably 1 IU/kg or more, still more preferably 1.5 IU/kg or more.

Preferably, IFN-γ is administered in an amount of 30 ng/kg body mass of a subject or less per administration dose to a skin of the subject. More preferably, IFN-γ is administered in an amount of 15 ng/kg or less, even more preferably of 10 ng/kg or less, still more preferably of 5 ng/kg or less, still even more preferably 2.5 ng/kg or less, furthermore preferably 1.5 ng/kg or less, most preferably 1 ng/kg or less. There is no lower limit for the amount per kg body mass as long as the technical effect can be achieved, however, usually, a lower limit is 0.025 ng/kg or more, more preferably 0.05 ng/kg or more, still more preferably 0.075 ng/kg or more.

Preferably, the IFN-γ is administered in a total amount of 30,000 IU or less, more preferably of 15,000 IU or less, even more preferably of 10,000 IU or less, still more preferably of 5,000 IU or less, still even more preferably 2,500 IU or less, furthermore preferably 1,500 IU or less, still furthermore preferably 1000 IU or less, still furthermore preferably 300 IU or less. There is no lower limit for the total amount as long as the technical effect can be achieved, however, usually, a lower limit is 2 IU or more, more preferably 10 IU or more.

Preferably, the IFN-γ is administered in a total amount of 1,500 ng or less, more preferably of 750 ng or less, even more preferably of 500 ng or less, still more preferably of 250 ng or less, still even more preferably 125 ng or less, furthermore preferably 75 ng or less, most preferably 50 ng or less. There is no lower limit for the total amount as long as the technical effect can be achieved, however, usually, a lower limit is 0.1 ng or more, more preferably 0.5 ng or more.

The amount per kg body mass and/or the total amount may be administered in one administration dose or distributed to a plurality of administration doses as long as the amounts are administered within 5 h or less, more preferably within 3 h or less, even more preferably within 1 h or less, still more preferably within 0.5 h or less, even further preferred within 15 min or less, still even further preferably within 5 min or less, most preferably, within 1 min or less in relation to step (A). In case of a plurality of administration doses, these may be administered separately, successively, together and/or simultaneously, wherein for the latter step (B) might be repeated.

Preferably, the amount per kg body mass and/or the total amount are administered at a frequency of once per day or less often, more preferably once per 2 days or less often, even more preferably once per 3 days or less often, still more preferably once per 4 days or less often, still even more preferably once per 5 days or less often, furthermore preferably once per week or less often, still furthermore preferably once per 2 weeks or less often, most preferably once per month or less often. Usually, there is no upper limit for the frequency as long the technical effect can be achieved, however, an upper limit may be, preferably, is once per two months or more often, more preferably, once per 6 weeks or more often. Preferably, the , steps (A) and (B), preferably, steps (A), (B) and(C) performed at a frequency of once per day to one per two months, more preferably once per 3 days to once per 6 weeks, even more preferably once per 5 days to once per month.

Preferably, the administrating of the IFN-γ to the skin may be, preferably, is accomplished by a topical administration and/or by injection, more preferably by a topical administration.

In case of a topical administration, this is preferably placed on the upper arm, forearm and/or upper leg.

In case of an injection, the injection is preferably into the sub-topical layers, more preferably, into the dermis and subcutis, most preferably into the subcutis and/or the injection is preferably placed 6 mm or less to 1 mm or more, more preferably 5 mm or less to 1 mm or more, even more preferably 4 mm or less to 2 mm or more in skin thickness direction. Preferably, the injection is placed into the upper arm, forearm and/or upper leg.

Preferably, in step (B), the skin are is a skin area [b] and, if applicable, the application area is an application area [b].

Preferably, the skin area [b] and the application area [b] are *mutatis mutandis* defined as the skin area [a] and the application area [a], respectively.

### Regarding optional step (C)

As already stated above, the method/the method of the medical use according to the present invention preferably comprises the further step of:
(C)administering a second immunomodulatory substance, preferably IL-2 to the skin of the subject.

Preferably, IL-2 is administered by administering the IL-2 or the composition comprising IL-2.

Generally, 1 ng IL-2 corresponds to 16.4 IU IL-2 or less. Some of the IL-2-protein might be denatured or otherwise defect in that the biological activity of IL-2 has been lost while the mass remains constant.

IL-2 has the technical effect of synergistically prolonging, enhancing and/or boosting the technical effect of IFN-γ. As seen from the examples, the administration of IL-2 alone without the administration of IFN-γ does not allow for achieving the technical effect. However, IL-2 can be harmful to a subject's body health in higher concentrations, hence, the administered amount should be kept as low as possible.

Preferably, IL-2 is administered in an amount of 10 IU/kg body mass of a subject or less per administration dose to a skin of the subject. More preferably, IL-2 is administered in an amount of 8 IU/kg or less, even more preferably of 5 IU/kg or less, most preferably of 4 IU/kg or less. There is no lower limit for the amount per kg body mass as long as the technical effect can be achieved, however, usually, a lower limit is 1 IU/kg or more, more preferably 2 IU/kg or more.

Preferably, IL-2 is administered in an amount of 0.6 ng/kg body mass of a subject or less per administration dose to a skin of the subject. More preferably, IL-2 is administered in an amount of 0.5 ng/kg or less, even more preferably of 0.3 ng/kg or less, most preferably of 0.25 ng/kg or less. There is no lower limit for the amount per kg body mass as long as the technical effect can be achieved, however, usually, a lower limit is 0.06 ng/kg or more, more preferably 0.12 ng/kg or more.

Preferably, the IL-2 is administered in a total amount of 500 IU or less, more preferably of 400 IU or less, even more preferably of 300 IU or less, most preferably of 200 IU or less. There is no lower limit for the total amount as long as the technical effect can be achieved, however, usually, a lower limit is 50 IU or more, more preferably 100 IU or more.

Preferably, the IL-2 is administered in a total amount of 30.5 ng or less, more preferably of 24.5 ng or less, even more preferably of 18.3 ng or less, most preferably of 12.2 ng or less. There is no lower limit for the total amount as long as the technical effect can be achieved, however, usually, a lower limit is 3 ng or more, more preferably 6.1 ng or more.

The amount per kg body mass and/or the total amount may be administered in one administration dose or distributed to a plurality of administration doses as long as the amounts are administered within 5 h or less, more preferably within 3 h or less, even more preferably within 1 h or less, still more preferably within 0.5 h or less, even further preferred within 15 min or less, still even further preferably within 5 min or less, most preferably, within 1 min or less. In case of a plurality of administration doses, these may be administered separately, successively, together and/or simultaneously, wherein for the latter step (B) might be repeated.

Preferably, the amount per kg body mass and/or the total amount are administered at a frequency of once per day or less often, more preferably once per 2 days or less often, even more preferably once per 3 days or less often, still more preferably once per 4 days or less often, still even more preferably once per 5 days or less often, furthermore preferably once per week or less often, still furthermore preferably once per 2 weeks or less often, most preferably once per month or less often. Usually, there is no upper limit for the frequency as long the technical effect can be achieved, however, an upper limit may be, preferably, is once per two months or more often, more preferably, once per 6 weeks or more often. Preferably, the , steps (A) and (B), preferably, steps (A), (B) and(C) performed at a frequency of once per day to one per two months, more preferably once per 3 days to once per 6 weeks, even more preferably once per 5 days to once per month.

Preferably, the administrating of the IL-2 to the skin may be, preferably, is accomplished by a topical administration and/or by injection, more preferably by a topical administration.

In case of a topical administration, this is preferably placed on the upper arm, forearm and/or upper leg.

In case of an injection, the injection is preferably into the sub-topical layers, more preferably, into the dermis and subcutis, most preferably into the subcutis and/or the injection is preferably placed 6 mm or less to 1 mm or more, more preferably 5 mm or less to 1 mm or more, even more preferably 4 mm or less to 2 mm or more in skin thickness direction. Preferably, the injection is placed into the upper arm, forearm and/or upper leg.

Preferably, in step (C), the skin are is a skin area [c] and, if applicable, the application area is an application area [c].

Preferably, the skin area [c] and the application area [c] are *mutatis mutandis* defined as the skin area [a] and the application area [a], respectively.

### Regarding steps (A), (B) and (C)

As already stated above, preferably, the skin areas [a] and [b] and, if applicable, [c] cover at least some skin, which is spatially distanced to and *immunological inactive and*/*or unchallenged skin.*

More preferably, the antigen free skin areas [a] and [b] and, if applicable, [c] overlap and/or are congruent to each other, in the following termed *"overlapping skin area",* or particularly *"overlapping skin area [a]*/*[b]*", *"overlapping skin area [b]*/*[c]"* and *"overlapping skin area [a]*/*[b]*/*[c]*, respectively.

A site of inflammation may be, preferably, is any site where an inflammation is present, for instance an inflamed joint, inflamed organ, allergic site, a wound and/or insect bite and it may be, preferably, is immunologically or auto-immunologically. More preferably, the special distance of the overlapping skin area from the site of inflammation is 0.5 cm or more, more preferably 1 cm or more, even more preferably 3 cm or more, still more preferably, 5 cm or more and most preferably 10 cm or more. There is no upper limit for such distance, however, usually, it is not more than 1.5 m, more preferably 1 m. The distance is measures from a point on the outline of the overlapping skin area, which lies closest to a site of inflammation. In case several sites of inflammation are present, the overlapping skin area is preferably located spatially distanced to and free to all these sites of inflammation.

The overlapping skin area [a]/[b] may have any size as long as the technical effect can be achieved and may have a size of even about 1 m² or less. Preferably, the skin area is 1000 cm² or less, more preferably 500 cm² or less, even more preferably 100 cm² or less, still more preferably 50 cm² or less, still even more preferably 25 cm² or less, still further preferably 10 cm², most preferably 5 cm² or less. Usually, the skin area is not smaller than 0.5 cm². Hence, a lower limit for the skin area is preferably 0.5 cm² or more, more preferably 1 cm² or more, still more preferably 2 cm² or more.

The overlapping skin area [b]/[c] may have any size as long as the technical effect can be achieved and may have a size of even about 1 m² or less. Preferably, the skin area is 1000 cm² or less, more preferably 500 cm² or less, even more preferably 100 cm² or less, still more preferably 50 cm² or less, still even more preferably 25 cm² or less, still further preferably 10 cm², most preferably 5 cm² or less. Usually, the skin area is not smaller than 0.5 cm². Hence, a lower limit for the skin area is preferably 0.5 cm² or more, more preferably 1 cm² or more, still more preferably 2 cm² or more.

The overlapping skin area [a]/[b]/[c] may have any size as long as the technical effect can be achieved and may have a size of even about 1 m² or less. Preferably, the skin area is 1000 cm² or less, more preferably 500 cm² or less, even more preferably 100 cm² or less, still more preferably 50 cm² or less, still even more preferably 25 cm² or less, still further preferably 10 cm², most preferably 5 cm² or less. Usually, the skin area is not smaller than 0.5 cm². Hence, a lower limit for the skin area is preferably 0.5 cm² or more, more preferably 1 cm² or more, still more preferably 2 cm² or more.

Preferably, steps (A) and (B), preferably, steps (A), (B) and(C) are performed within 5 h or less, more preferably within 3 h or less, even more preferably within 1 h or less, still more preferably within 0.5 h or less, even further preferred within 15 min or less, still even further preferably within 5 min or less, most preferably, within 1 min or less.

Preferably, steps (A) and (B), preferably, steps (A), (B) and(C) performed at a frequency of once per day or less often, more preferably once per 2 days or less often, even more preferably once per 3 days or less often, still more preferably once per 4 days or less often, still even more preferably once per 5 days or less often, furthermore preferably once per week or less often, still furthermore preferably once per 2 weeks or less often, most preferably once per month or less often. Usually, there is no upper limit for the frequency as long the technical effect can be achieved, however, an upper limit may be, preferably, is that steps (A) and (B), preferably, steps (A), (B) and(C) performed at a frequency of once per two months or more often, more preferably, once per 6 weeks or more often.

Preferably, the , steps (A) and (B), preferably, steps (A), (B) and(C) performed at a frequency of once per day to one per two months, more preferably once per 3 day to once per 6 weeks, even more preferably once per 5 days to once per month.

Furthermore, the present invention further relates to a kit of parts comprising
i) a means for generating an accumulation of PBMCs within the skin of a subject and
ii) an immunomodulatory substance as described in any one of the embodiments described herein.

The means for generating an accumulation of PBMCs within the skin of a subject preferably is any means as described in A-1 to A-10. For example, in case of, the heat is applied by any means known to the person skilled in the art which do not cause lesions and/or a damaging of skin as described in A-5. For instance, the heat is applied by using heating means like radiation like infrared radiation, moxibutation, hyperthermia, manual skin massage using vibration and/or rubbing of the skin, a warm object like a heater, a heat pad or a preheated object, a hot air, warm tea cup, etc.

Furthermore, for example in case of a blood-circulation-increasing agent, such agent is any substance, composition or formulation suitable to dilate the vessels and capillaries, particularly the capillaries, of the skin.

Preferably, the kit of parts further comprises
iii) a second immunomodulatory substance as described in any one of the embodiments described herein.

Furthermore, the present invention relates to a kit of parts comprising
i) an immunomodulatory substance in any one of the embodiments described herein and
ii) a second immunomodulatory substance in any one of the embodiments described herein.

Preferably, the kit of parts further comprises
iii) a means for generating an accumulation of PBMCs within the skin of a subject.

Preferably, the kit of parts as described in any one of the embodiments described herein is for use in a method for treating and/or preventing inflammations and /or inflammatory diseases in any one of the embodiments as described herein.

The substances as used in the present invention may be administered by any suitable route of administration to the skin. Preferably, the substances are prepared for and administered by topical administration or injection, for example as described herein. A topical application includes every administration that is applied to a particular place on or in the body, for example an application to body surfaces such as the skin. Topical administrations include epicutaneous application, meaning that they are applied directly to the skin. For topical application range of classes including creams, foams, gels, emulsions, powders, liquid or solid preparations for inhalation, compresses, tamponades, tonsil pinhole solutions or gargle solutions lotions, sprays and ointments may be used.

The substances as described in any one of the embodiments herein are usually formulated into compositions, preferably pharmaceutical compositions. Hence, in a further aspect, the present invention further relates to compositions comprising the substances in any one of the embodiments as described herein as an active ingredient, optionally together with a suitable carrier substance and/or excipient. Such pharmaceutical compositions comprise an effective amount of the substances, preferably as described in any one of the embodiments as described herein.

Accordingly, such pharmaceutical compositions may be for use in the methods for treating and/or preventing diseases in any one of the embodiments as described herein.

The pharmaceutical compositions may optionally further comprise one or more additional pharmaceutically active compounds. For example, mixtures of the immunomodulatory substances as described herein may be used. Further, pharmaceutically acceptable excipient meaning a pharmaceutically acceptable material, composition or vehicle involved in giving form or consistency to the pharmaceutical composition may be present in the pharmaceutical compositions.

In particular, the pharmaceutical compositions and or dosage forms may be in the form of slow-release formulations, ointments, emulsions, particulate material, powders, liquid or solid preparations for inhalation, compresses, wadding, tamponades, creams, foams, gels, sprays, tonsil pinhole solutions, plaster, bandage and/or pads. The pharmaceutical preparation may also be a dermatological preparation, preferably in the form of an application for external use.

Preferably, the topical dosage form and/or the dosage form suitable for injection further comprises a immunomodulatory substance as described in any one of the embodiments described herein.

Preferably, the topical dosage form and/or the dosage form suitable for injection further comprises a substance for generating an accumulation of PBMCs within the skin of a subject.

Preferably, the topical dosage form and/or the dosage form suitable for injection further comprises a second immunomodulatory substance.

Preferably, the pharmaceutical dosage form is a plaster, pad or bandage. More preferably, the plaster, pad or bandage further comprises any one of the immunomodulatory substances disclosed in step (B). Even more preferably, the plaster, pad or bandage is suitable to deliver the same amount of IFN-γ as preferably administered in step (B) to the skin of the subject. Preferably, the delivery is into the dermis and/or subcutis of the skin, preferably the skin area.

More preferably, the plaster, pad or bandage comprises IFN-γ in a total amount of 30.000 IU (about 1.500 ng/ml) or less.

Furthermore, preferably, the plaster, pad or bandage further comprises any one of the substance disclosed in step (A).

Furthermore, preferably, the plaster, pad or bandage further comprises any one of the immunomodulatory substances disclosed in step (C). More preferably, the plaster, pad or bandage further IL-2. Preferably, the plaster, pad or bandage is suitable to deliver the same amount of IL-2 as preferably administered in step (C) to the skin of the subject. Preferably, the delivery is into the dermis and/or subcutis of the skin, preferably the skin area.

Hence, more preferably, the plaster, pad or bandage comprises any one of the immunomodulatory substances disclosed in step (B) and (C), preferably IFN-γ and IL-2 in any one of the embodiments as described herein.

Furthermore, the dosage form suitable for injection is a cartridge for an injection pen, carpule and/or syringe. More preferably, the cartridge for an injection pen, carpule and/or syringe further comprises any one of the immunomodulatory substances disclosed in step (B). Even more preferably, the cartridge for an injection pen, carpule and/or syringe is suitable to deliver the same amount of IFN-γ as preferably administered in step (B) to the skin of the subject. Preferably, the delivery is into the dermis and/or subcutis of the skin, preferably the skin area.

The dosage form suitable for injection comprising IFN-γ is any dosage form that allows for an administration of IFN-γ per injection.

Preferably, the dosage form suitable for injection comprising IFN-γ is a cartridge for an injection pen, carpule and/or syringe, filled with a composition comprising IFN-γ.

Preferably, the cartridge for an injection pen, carpule and/or syringe, filled with a composition comprising IFN-γ is suitable for use in the method.

Preferably, the cartridge for an injection pen, carpule and/or syringe, filled with a composition comprising IFN-γ in an amount of 50.000 IU/ml or less, preferably 10.000 IU/ml or less, or 2.500 ng/ml or less, preferably 500 ng/ml or less.

Furthermore, preferably, cartridge for an injection pen, carpule and/or syringe further comprises any one of the substance disclosed in step (A).

Furthermore, preferably, the cartridge for an injection pen, carpule and/or syringe further comprises any one of the immunomodulatory substances disclosed in step (C). More preferably, the cartridge for an injection pen, carpule and/or syringe further comprises IL-2. Preferably, the cartridge for an injection pen, carpule and/or syringe is suitable to deliver the same amount of IL-2 as preferably administered in step (C) to the skin of the subject. Preferably, the delivery is into the dermis and/or subcutis of the skin, preferably the skin area.

Hence, more preferably, the cartridge for an injection pen, carpule and/or syringe comprises any one of the immunomodulatory substances disclosed in step (B) and (C), preferably IFN-γ and IL-2 in any one of the embodiments as described herein.

Furthermore, the present invention relates to a composition comprising a substance for generating an accumulation of PBMCs within the skin of a subject as described in any one of the embodiments as herein and an immunomodulatory substance as described in any one of the embodiments as herein.

Preferably, the composition further comprises a second immunomodulatory substance as described in any one of the embodiments as herein.

Furthermore, the present invention relates to a topical dosage form, preferably plaster, pad or bandage comprising an immunomodulatory substance as described in any one of the embodiments described herein.

Preferably, the plaster, pad or bandage comprising the immunomodulatory substance, wherein the plaster, pad or bandage is suitable to deliver an amount of 30000 IU or less or about 1500 ng or less an immunomodulatory substance or less subcutaneously and/or intracutaneously to a skin area.

Furthermore, the present invention relates to a composition for topical application and/or injection, comprising IFN-γ in an amount of 50.000 IU/ml or less or 2.500 ng/ml or less.

Preferably, the composition for topical application and/or injection, comprising IFN-γ in an amount of 15.000 IU/ml or less or 750 ng/ml or less.

Preferably, the composition for topical application, comprising IFN-γ in an amount of 50.000 IU/ml or less or about 2.500 ng/ml or less.

Composition for topical application and/or injection, comprising IFN-γ and IL-2.

### FURTHER EMBODIMENTS OF THE PRESENT INVENTION

The present invention also relates to:
- a topical dosage form comprising an immunomodulatory substance; and/or
- a dosage form suitable for injection comprising an immunomodulatory substance;
preferably in a method for treating and/or preventing an inflammatory disease, wherein the method comprises the steps of:
(A)generating an accumulation of PBMCs within the skin of a subject; and
(B)administering the immunomodulatory substance to the skin of said subject, by applying the topical dosage form to the skin and/or by performing an injection into the skin by using the dosage form suitable for injection;
and to the method as such.

In a particularly preferred embodiment the present invention relates to:
- a topical dosage form comprising IFN-γ: and/or
- a dosage form suitable for injection comprising IFN-γ:
preferably in a method for treating and/or preventing an inflammatory disease, wherein the method comprises the steps of:
(A) generating an accumulation of PBMCs within the skin of a subject; and
(B)administering IFN-γ to the skin of said subject, by applying the topical dosage form to the skin and/or by performing an injection into the skin by using the dosage form suitable for injection;
and to the method as such.

As mentioned above in an aspect of all embodiments of the invention, the method further may comprise the step of:
(C) administering a second immunomodulatory substance to the skin.

The second immunomodulatory substance administered in step (C) is different from said first immunomodulatory substance administered in step (B).

The second immunomodulatory substance administered in step (C) may be selected from any one of the embodiments of the immunomodulatory substance as described herein. In a particularly preferred embodiment, as mentioned above, the second immunomodulatory substance administered in step (C) comprises or consists of IL-2.

Administering the second immunomodulatory substance to the skin of a subject in step (C) may be carried out by administering the immunomodulatory substance as such or as a component of a composition, i.e. as a composition comprising the immunomodulatory substance.

Such a composition may also comprise both immunomodulatory substances to be administered in steps (B) and (C).

Furthermore, administering the immunomodulatory substance to the skin of a subject in step (C) may involve to administer the immunomodulatory substance in a topical dosage form comprising the immunomodulatory substance, and/or to administer the immunomodulatory substance in a dosage form suitable for injection comprising the immunomodulatory substance.

Such a topical dosage form and/or dosage form suitable for injection may also comprise both immunomodulatory substances to be administered in steps (B) and (C).

This applies to any one of the embodiments of the invention described herein involving steps (B) and (C).

The present invention also relates to:
- a first immunomodulatory substance;
- a second immunomodulatory substance;
for use in a method for treating and/or preventing an inflammatory disease, wherein the method comprises the steps of:
(A)generating an accumulation of PBMCs within the skin of a subject;
(B)administering the first immunomodulatory substance to the skin of said subject; and
(C)administering the second immunomodulatory substance to the skin of said subject;
and to said method as such.

In a preferred embodiment the invention relates to:
- IFN-γ:
- a second immunomodulatory substance;
for use in a method for treating and/or preventing an inflammatory disease, wherein the method comprises the steps of:
(A)generating an accumulation of PBMCs within the skin of a subject;
(B)administering IFN-γ to the skin of said subject; and
(C)administering the second immunomodulatory substance to the skin of said subject;
and to said method as such.

Still further preferred the invention relates to:
- IFN-γ:
- IL-2;
for use in a method for treating and/or preventing an inflammatory disease, wherein the method comprises the steps of:
(A)generating an accumulation of PBMCs within the skin of a subject;
(B)administering IFN-γ to the skin of said subject; and
(C)administering IL-2 to the skin of said subject;
and to said method as such.

The present invention further relates to:
- a topical dosage form comprising a first immunomodulatory substance;
- a topical dosage form comprising a second immunomodulatory substance;
- a topical dosage form comprising the first immunomodulatory substance and second immunomodulatory substance;
- a dosage form suitable for injection comprising the first immunomodulatory substance; and/or
- a dosage form suitable for injection comprising the second immunomodulatory substance
- a dosage form suitable for injection comprising the first immunomodulatory substance and the second immunomodulatory substance,
preferably in a method for treating and/or preventing inflammatory diseases, immunological diseases and autoimmunological diseasess in any one of the embodiments as described herein, preferably in a method wherein the method comprises the steps of:
(A)generating an accumulation of lymphocytes within skin of a subject;
(B)applying the first immunomodulatory substance to the skin of the subject by applying one of the topical dosage forms comprising the first immunomodulatory substance to the skin and/or by performing an injection into the skin by using one of the dosage form suitable for injection comprising the first immunomodulatory substance; and
(C)applying the second immunomodulatory substance to the skin of the subject by applying one of the topical dosage forms comprising the second immunomodulatory substance to the skin and/or by performing an injection into the skin by using one of the dosage form suitable for injection comprising the second immunomodulatory substance.

In a preferred embodiment, the present invention relates to:
- a topical dosage form comprising IFN-γ:
- a topical dosage form comprising a second immunomodulatory substance;
- a topical dosage form comprising IFN-γ and second immunomodulatory substance;
- a dosage form suitable for injection comprising IFN-γ: and/or
- a dosage form suitable for injection comprising the second immunomodulatory substance
- a dosage form suitable for injection comprising IFN-γ and the second immunomodulatory substance,
preferably in a method for treating and/or preventing inflammatory diseases, immunological diseases and autoimmunological diseasess in any one of the embodiments as described herein, preferably in a method wherein the method comprises the steps of:
(A)generating an accumulation of lymphocytes within skin of a subject;
(B)applying IFN-γ to the skin of the subject by applying one of the topical dosage forms comprising IFN-γ to the skin and/or by performing an injection into the skin by using one of the dosage form suitable for injection comprising IFN-γ: and
(C)applying the second immunomodulatory substance to the skin of the subject by applying one of the topical dosage forms comprising the second immunomodulatory substance to the skin and/or by performing an injection into the skin by using one of the dosage form suitable for injection comprising the second immunomodulatory substance.

In a still a further preferred embodiment, the present invention relates to:
- a topical dosage form comprising IFN-γ:
- a topical dosage form comprising IL-2;
- a topical dosage form comprising IFN-γ and IL-2;
- a dosage form suitable for injection comprising IFN-γ: and/or
- a dosage form suitable for injection comprising IL-2;
- a dosage form suitable for injection comprising IFN-γ and IL-2,
preferably in a method for treating and/or preventing inflammatory diseases, immunological diseases and autoimmunological diseasess in any one of the embodiments as described herein, preferably in a method wherein the method comprises the steps of:
(A)generating an accumulation of lymphocytes within skin of a subject;
(B)applying IFN-γ to the skin of the subject by applying one of the topical dosage forms comprising IFN-γ to the skin and/or by performing an injection into the skin by using one of the dosage form suitable for injection comprising IFN-γ: and
applying IL-2 to the skin of the subject by applying one of the topical dosage forms comprising IL-2 to the skin and/or by performing an injection into the skin by using one of the dosage form suitable for injection comprising IL-2.

The present invention also relates to an immunomodulatory substance for use in a method for treating and/or preventing an inflammatory disease, wherein the method comprises the steps of:
(A-1) generating a vasodilation of the capillaries within the skin of a subject; and
(B) administering the immunomodulatory substance to the skin of said subject; and to said method as such.

In this embodiment of the invention, all preferred embodiments as described herein above for (A-1) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory disease, wherein the method comprises the steps of:
(A-2) generating an increased blood flow within the skin of a subject, wherein the blood flow is preferably increased by 1% or more, preferably when measured as described in the methods measurement of the blood flow, and/or is increased by 10 µl/s or more, preferably when measured as described in the methods measurement of the blood flow; and
(B) administering the immunomodulatory substance to the skin of said subject; and to said method as such.

In this embodiment of the invention, all preferred embodiments as described herein above for (A-2) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory disease, wherein the method comprises the steps of:
(A-3) generating an increased temperature on the skin of a subject, wherein preferably the temperature is increased by 1 % or more and/or is increased by 0.2 °C or more, preferably when measured as described in the methods measurement of the temperature of the skin; and
(B) administering the immunomodulatory substance to the skin of said subject; and to said method as such.

In this embodiment of the invention, all preferred embodiments as described herein above for (A-3) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory disease, wherein the method comprises the steps of:
(A-4) generating a redness on the skin of a subject, wherein the redness is visually detectable to the naked eye; and
(B) administering the immunomodulatory substance to the skin of said subject; and to said method as such.

In this embodiment of the invention, all preferred embodiments as described herein above for (A-4) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory disease, wherein the method comprises the steps of:
(A-5) applying heat to the skin of a subject; and
(B) administering the immunomodulatory substance to the skin of said subject; and to said method as such.

In this embodiment of the invention, all preferred embodiments as described herein above for (A-5) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory disease, wherein the method comprises the steps of:
(A-6) applying mechanical agitation to the skin of a subject; and
(B) administering the immunomodulatory substance to the skin of said subject; and to said method as such.

In this embodiment of the invention, all preferred embodiments as described herein above for (A-6) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory disease, wherein the method comprises the steps of:
(A-7) applying negative pressure to the skin of a subject; and
(B) administering the immunomodulatory substance to the skin of said subject; and to said method as such.

In this embodiment of the invention, all preferred embodiments as described herein above for (A-7) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory disease, wherein the method comprises the steps of:
(A-8) administering a blood-circulation-increasing agent to the skin of a subject; and
(B) administering the immunomodulatory substance to the skin of said subject; and to said method as such.

In this embodiment of the invention, all preferred embodiments as described herein above for (A-8) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory disease, wherein the method comprises the steps of:
(A-9) administering a vasodilating agent to the skin of a subject; and
(B) administering the immunomodulatory substance to the skin of said subject; and to said method as such.

In this embodiment of the invention, all preferred embodiments as described herein above for (A-9) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory disease, wherein the method comprises the steps of:
(A-10) injecting PBMCs into the skin of a subject, preferably the skin of the skin area; and
(B) administering the immunomodulatory substance to the skin of said subject; and to said method as such.

In this embodiment of the invention, all preferred embodiments as described herein above for (A-10) are also preferred, and, furthermore, also all preferred embodiments of the elements of the invention such as the inflammatory disease, the subject and the immunomodulatory substance as well as its administration as described herein are also preferred.

The present invention further relates to an immunomodulatory substance for use in a method for treating and/or preventing an inflammatory disease, immunological disease and/or autoimmunological disease, wherein the method comprises the steps of:
(A) generating an increased temperature on the skin within the skin of a subject; and
(B) administering the immunomodulatory substance to the skin of said subject.

In this embodiment of the invention, all preferred embodiments as described herein above for (A-3) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory diseases, immunological disease and/or autoimmunological disease, wherein the method comprises the steps of:
I. administering the immunomodulatory substance to the skin of a subject; wherein the amount administered does not cause an systemic increase of the concentration of the immunomodulatory substance of the subject
and to said method as such.

Preferably, the immunomodulatory substance is IFN-γ. More preferably, the immunomodulatory substance is IFN-γ and administered in an amount of 100 IU/kg body weight of a subject or less or 5 ng/ kg body weight a subject or less Preferably, the immunomodulatory substance is IFN-γ and administered in a total amount of 30.000 IU or less or 1500 ng or less.

All preferred embodiments as described herein above for (B) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory diseases, immunological disease and/or autoimmunological disease, wherein the method comprises the steps of:
I. administering the immunomodulatory substance to the skin of a subject; wherein the amount administered does not cause an systemic increase of the concentration of the immunomodulatory substance of the subject; and
II. administering the second immunomodulatory substance to the skin of a subject; wherein the amount administered does not cause an systemic increase of the concentration of the immunomodulatory substance of the subject
and to said method as such.

Preferably, step (I) and step (II) can be performed separately, successively, together and/or simultaneously.

Preferably, the immunomodulatory substance administered in I is IFN-γ and second immunomodulatory substance administered in II is IL-2.

All preferred embodiments as described herein above for (B) are also preferred, and, furthermore, also all preferred embodiments of the elements of the invention such as the inflammatory disease, the subject and the immunomodulatory substance as well as its administration as described herein are also preferred. All preferred embodiments as described herein above for (C) are also preferred, and, furthermore, also all preferred embodiments of the elements of the invention such as the inflammatory disease, the subject and the immunomodulatory substance as well as its administration as described herein are also preferred.

The present invention also relates to an immunomodulatory substance for use in a method for treating and/or preventing an inflammatory diseases, immunological disease and/or autoimmunological disease, wherein the method comprises the steps of:
I. topically administering IFN-γ to the skin area of a subject distanced to a site of immunological activity or challenge
and to said method as such.

All preferred embodiments as described herein above for (B) are also preferred, and, furthermore, also all preferred embodiments of the elements of the invention such as the inflammatory disease, the subject and the immunomodulatory substance as well as its administration as described herein are also preferred.

Furthermore, the present invention further relates to a kit of parts comprising
i) a means for generating an accumulation of PBMCs within the skin of a subject and
ii) an immunomodulatory substance as described in any one of the embodiments described herein.

All preferred embodiments as described herein above for (A) and (B) are also preferred, and, furthermore, also all preferred embodiments of the elements of the invention such as the inflammatory disease, the subject and the immunomodulatory substance as well as its administration as described herein are also preferred.

Furthermore, the present invention relates to a kit of parts comprising
i) an immunomodulatory substance in any one of the embodiments described herein and
ii) a second immunomodulatory substance in any one of the embodiments described herein.

All preferred embodiments as described herein above for (B) and (C) are also preferred, and, furthermore, also all preferred embodiments of the elements of the invention such as the inflammatory disease, the subject and the immunomodulatory substance as well as its administration as described herein are also preferred.

Furthermore, the present invention relates to a plaster, pad or bandage comprising IFN-γ as described in any one of the embodiments described herein.

Furthermore, the present invention relates to cartridge for an injection pen, carpule and/or syringe comprising IFN-γ as described in any one of the embodiments described herein.

Furthermore, the present invention relates to a composition for topical application, comprising IFN-γ as described in any one of the embodiments described herein and a blood-circulation-increasing substance as described herein above for (A-8).

Furthermore, the present invention relates to a composition for topical application, comprising IFN-γ as described in any one of the embodiments described herein and IL-2 as described in any one of the embodiments described herein.

Furthermore, the present invention relates to a composition for topical application and/or injection, comprising IFN-γ as described in any one of the embodiments described herein in an amount of 50.000 IU/ml or less or 2.500 ng/ml or less.

### Disclaimers and further preferred embodiments

Preferably, the method does not comprise administering, neither topically, neither by injection and/or neither by any other way, of cantharidin in a range of no cantharidin is administered up to an amount of 1 mg or less is administered.

Preferably, the IFN-γ is not the subject's endogenous IFN-γ. Hence, preferably, the method does not comprise administering, neither topically, neither by injection and/or neither by any other way, of the subject's endogenous IFN-γ in a range of no subject's endogenous IFN-γ is administered up to an amount of 30.000 or less is administered.

Preferably, the method does not comprise administering, neither topically, neither by injection and/or neither by any other way, of IL-6 (interleukin 6), particularly not of subject's endogenous IL-6, in a range of no IL-6 is administered up to an amount of 2000 IU or less is administered.

Preferably, the method does not comprise administering, neither topically, neither by injection and/or neither by any other way, of insulin in a range of no insulin is administered up to an amount of 500 IU or less is administered.

Preferably, the method does not comprise administering, neither topically, neither by injection and/or neither by any other way, of exudate, lymph fluid, wound fluid and/or blister fluid like blister fluid extracted from a blister caused by cantharidin, in a range of no subject's endogenous IFN-γ is administered up to a total amount of 10 ml or less is administered. Preferably, the of exudate, lymph fluid, wound fluid and/or blister fluid like blister fluid extracted from a blister caused by cantharidin, is the subject's endogenous exudate, lymph fluid, wound fluid and/or blister fluid.

Preferably, the method does not comprise administering, preferably in steps (B) and/or (C), neither topically, neither by injection and/or neither of any substances as active ingredients which are not similar or identical to any protein or fragment thereof naturally occurring in a vertebrate, preferably a mammal, more preferably a subject as defined below More preferably, the method does not comprise administering, preferably in steps (B) and/o (C), neither topically, neither by injection and/or neither of any substances as active ingredients other than cytokines, still more preferably, one or more immune-related cytokines.

Preferably, the method does not comprise administering platelets in a range of no platelets are administered.

Disclaimer, overlapping are must not be located closer than....inflammation area.

### BRIEF DESCRIPTION OF THE FIGURES

Fig. 1 shows a graph of the initial value and development of the amount of CRP over a treatment course of 21 weeks of one patient suffering from rheumatoid arthritis (patient 01), including controls, placebo, incorrect treatment and treatment according to the present invention.
Fig. 2 shows a graph of the initial value and the development of the relative CRPs of three patients suffering from rheumatoid arthritis (patients 01, 02 and 04) over of up to 5 weeks upon receiving a treatment according to the present invention.
Fig. 3 shows a bar chart of the development of the relative CRPs of three patients suffering from rheumatoid arthritis (patients 01, 02 and 04) upon receiving treatment according to the present invention.
Fig. 4 shows a bar chart of the development of the relative CRP of one patient suffering from polyarthritis (patient 05) upon receiving treatment according to the present invention.
Fig. 5 shows photographs of the right and left hand of a patient suffering from rheumatoid arthritis (patient 02), wherein week 0 depicts the initial condition and weeks 1 to 3 the improvement upon receiving treatment according to the present invention.

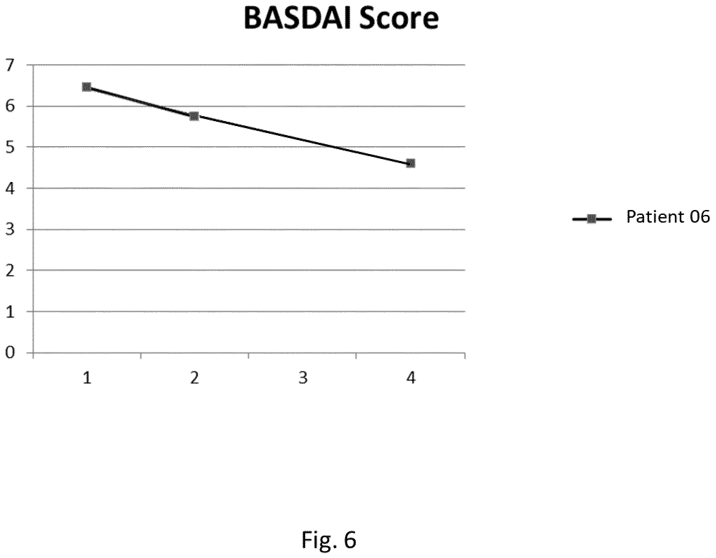

Fig. 6 shows a graph of the development of the BASDAI of one patient suffering from ankylosing spondylitis [Morbus Bechterew] (patient 06) upon receiving treatment according to the present invention.

### MATERIALS AND METHODS

### 1. Measurement of the vasodilation

Measured according to a method known to the person skilled in the art.

### 2. Measurement of accumulation of lymphocytes

Measured according to a method known to the person skilled in the art.

### 3. Measurement of the blood flow

(A) The effect of local warming on the blood flow was described by Minson CT *et al.* (Minson CT, Berry LT, Joyner MJ. Nitric oxide and neurally mediated regulation of skin blood flow during local heating. J Appl Physiol (1985). 2001 Oct;91 (4):1619-26. ) where a detailed description can be found. In short Skin blood flow (SkBF) of one forarm was measured. SkBF was recorded for 30 min to serve as a baseline. During the baseline period, the temperature of the local heating units at the microdialysis sites was kept constant at 33°C. After baseline, temperature of the local heating units was increased at a rate of 0.5°C every 5 s to a temperature of 42°C. The local heating units were held constant at 42°C for 50-80 min.

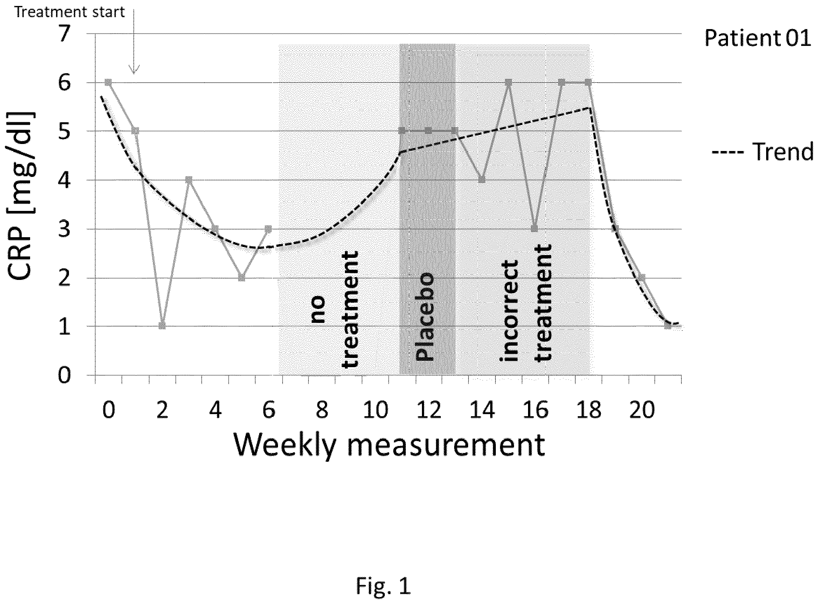

   Figure 1A of Minson CT *et al.* is a representative tracing of the temperature responses to the local heating protocol. At the initiation of heating, skin temperature at the heating probe-skin surface interface rapidly increased to 39-39.5°C and remained stable throughout the heating protocol. Figure 1B of Minson CT *et al.* is a representative tracing of the SkBF response to the local heating protocol. After the baseline period (33°C), the local heater temperature was increased to 42°C, resulting in a rapid increase in SkBF to an initial peak (average +/- standard error (SE) for all subjects: 75 +/- 3% SkBFmax). This was followed by a transient drop in SkBF (56 +/- 3% SkBFmax), followed by a secondary progressive rise to a plateau (87 +/- 4% SkBFmax).
(B) Furthermore or alternatively, the blood flow within the skin capillaries can be measured by O2C (oxygen to see). The O2C examination combines tissue photospectrometry (ATS) with laser Doppler flow measurement (LDA; Laser-Doppler-Anemometrie ) - O2C-LDA has been used in technical areas since the 1980s, but in medicine this method only became established in the 1990s and has so far only been used at a few centres. Laser Doppler Anemometry (LDA) is an optical measuring method for the punctual determination of velocity components in flows. A split laser beam crosses at the measuring point, where interference fringe patterns are created. A particle moving through the fringe pattern generates a scattered light signal whose frequency is proportional to the velocity component perpendicular to the interference fringes and is detected by the light detector. By combining laser Doppler systems with different laser wavelengths, all flow velocity components can be detected. In addition to capillary venous oxygen saturation and haemoglobin quantity, the flow velocity in the capillaries can be determined simultaneously (LEA Medizintechnik http://www.lea.de/deu/fro2chd.htm).

### 4. Measurement of temperature of the skin

Measured according to a method known to the person skilled in the art.

### 5. Preparation of PBMCs

All items and materials to be placed on the working place are extensively disinfected with 80 % ethanol (v/v in aqua ad injectabilia, Braun) prior to placing them on the working place. 30 µl sodium heparin (Heparin-Natrium supplied by Braun, B. Braun Melsungen AG, 25 000 I.E./5 ml (IU/5 ml) Injection solution, PZN:15782698) are transferred to a 10 ml syringe.

The syringe was equipped with a 21G butterfly injection needle and used to collect venous blood from the patient. The syringe was inverted three times in order to mix heparin and blood. The heparin blood was transferred to 10 ml sterile centrifugation tube. Two addition 10 ml sterile centrifugation tubes were equipped with 3 ml lymphocytes separation medium (Lymphocyte Separating Medium, Pancoll human, density: 1.077 g/ml, product number P04-60125). The lymphocytes separation medium was carefully overlaid with 5 ml of the heparin blood. These two additional 10 ml sterile centrifugation tubes were centrifuged for 15 min at 850 × g without break in order to allow separation of mononuclear leucocytes from the blood by density gradient centrifugation.

The white interface containing PBMCs was carefully harvested by pipetting and transferred in fresh 10 ml centrifugation tubes. These tubes were centrifuges for 12 min at 1900 × g. The supernatant was discarded and the cellular pellet solved in 10 ml sterile 0.9% NaCl (0.9% Braun Ecoflac Plus, B. Braun Melsungen AG, PZN: 08609255) and centrifuged for 8 min at 750 × g. The supernatant was discarded, the cellular pellet resuspended in 10 ml sterile 0.9% NaCl (0.9% Braun Ecoflac Plus, B. Braun Melsungen AG, PZN: 08609255) centrifuged for 8 min at 750 × g. The supernatant was discarded and the cellular pellet resuspended in 0.1 ml 0.9% NaCl (0.9% Braun Ecoflac Plus, B. Braun Melsungen AG, PZN: 08609255) and transferred to a 2 ml syringe.

### 6. Preparation of IFN-γ injection solution

From one Ampulle of Imukin^{®} Injektionslösung 2x 106 IU (0.1 mg) (PZN: 06958744, Lot: M000487) 500 µl were mixed with 199.5 ml 0.9% NaCl (Braun Ecoflac Plus, B. Braun Melsungen AG, PZN: 08609255). This resulted in a final concentration of 10,000 IU/ml. The solution was frozen in 500 µl aliquots in sterile glas ampulles and stored at -20°C until use.

Prior to injection, the substance was thawed at room temperature and 0.1 ml was injected in the patient (1000 IU).

If a lower concentration of IFN-γ was need, a dilution was prepared as followes:
One aliquot, 0.5 ml of the above described IFN-γ solution (10,000 IU/ml) was thawed and mixed with 4.5 ml 0.9% NaCl (Kochsalzlösung 0.9% Miniplasco connect, B. Braun Melsungen AG, PZN: 03079870). From this dilution, 0.1ml was used for treatment of the patient.

### 7. Preparation of IL-2 injection solution

One ampulle of Proleukin S (18 × 106 IU) was solved in 9 ml water for injection (AQUA AD Injectabilia Miniplasco connect, B. Braun Melsungen AG, PZN 03113087) to achieve a final concentration of 2 × 106 IU/ml. 0.4ml of this solved IL-2 was mixed with 399.6 ml 0.9% NaCl (Braun Ecoflac Plus PZN: 08609255). This resulted in a final concentration of 2,000 IU/ml. The solution was frozen in 500 µl aliquots in sterile glas ampulles.

Prior to injection, the substance was thawed at room temperature and 0.1 ml was injected in the patient (200 IU).

### 8. Preparation of ointment comprising IFN-γ

From one 500 µl Ampulle of Imukin^{®} Injektionslösung 2x 106 IU (0.1 mg) (PZN: 06958744, Lot: M000487) 500 µl were mixed with 19.5 ml 0.9% NaCl (Braun Ecoflac Plus, B. Braun Melsungen AG, PZN: 08609255). This resulted in a final concentration of 100,000 IU/ml.

9.44 g Base creme DAC (Basiscreme DAC, Bombastus-Werke AG, PZN 04193119; density 9.5 g/10ml) were mixed with 0.15 ml of the above prepared IFN-γ solution resulting in a concentration of 150 IU/100 µl.

The patient used 0.1 ml daily for topical application.

### 9. Vasodilation // increasing blood flow // increased the skin temperature // redness of the skin by using a heat pad

The skin was topically heated by using a heat pad with a size of 10 cm × 10 cm ("Relags Magic Heat Pad" by basic NATURE, BSN350974), developing according to the manufactures instructions a maximum temperature of 58°C. The heat pad was activated by pressing and bending the integrated chip-bottom. The activated heat pas was placed for 10 sec up to 5 min on the skin of the upper side of the patient's forearm. The skin became noticeably warm, as could be determined by touching it with the hand. Furthermore, in case of a caucasian skin, a clear redness of the skin developed, wherein the redness was visually detectable to the naked eye.

### 10. Vasodilation // increased blood flow // increased the skin temperature // redness of the skin by using creme

Heat creme (Kytta^{®} heat balm containing methylnicotinat ["Kytta^{®} Wärmebalsam" by P&G Health Germany GmbH, Germany, PZN 12358936]) was applied topically to the skin of the patient. The skin area, to which the heat creme was applied, was in the range of 4 cm² to 100 cm². About 50 mg to 800 mg heat creme were administered to the skin area by applying a thin film.

Methylnicotinat is known to act as vasodilating agent and causes an increase in the skin temperature as demonstrated under '3. Measurement of the blood flow'.

A detailed description of the effect of methyl nicotinate on the blood flow can be found in Tur E et al. (Tur E, Guy RH, Tur M, Maibach HI. Noninvasive assessment of local nicotinate pharmacodynamics by photoplethysmography. J Invest Dermatol. 1983 Jun;80(6):499-503.)

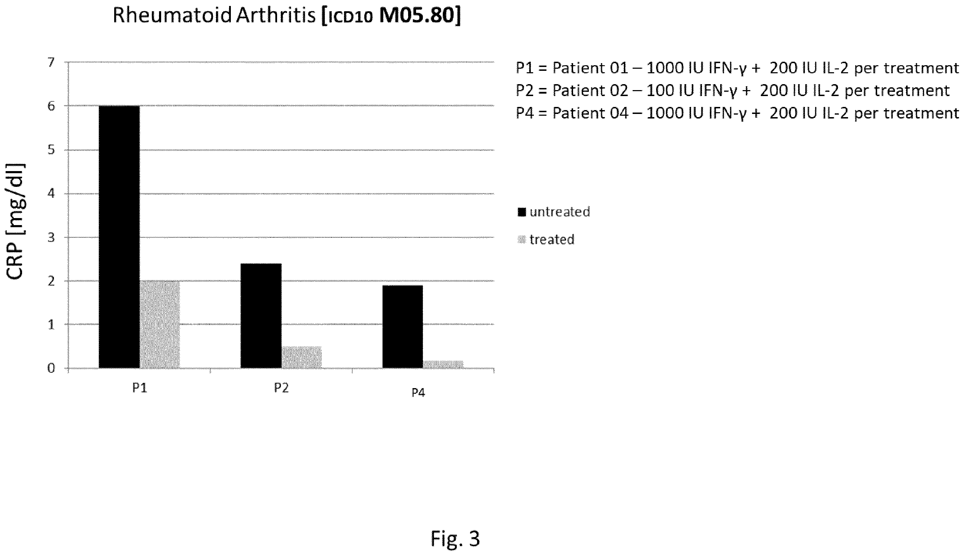

The local pharmacodynamics of a topical vasodilator (methyl nicotinate (NM) has been described by Elawa et al. (Elawa S, Mirdell R, Farnebo S, Tesselaar E. Skin blood flow response to topically applied methyl nicotinate: Possible mechanisms. Skin Res Technol. 2020 May;26(3):343-348. Epub 2019 Nov 27. PMID: 31777124.) Particularly, the authors described that a dose of 50 µl of 20 mmol/LMN results in a reproducible perfusion response in healthy test subjects using a Laser Speckle Contrast Imager (PeriCam PSI System; Perimed AB) was used to measure the perfusion of the skin. The perfusion increase occurs a few minutes after topical application of MN and reaches a stable plateau phase after five minutes. This plateau phase lasts for at least another 15 minutes (Elawa *et al.,* Figure 3).

After an initial decrease in the skin temperature due to evaporative cooling the skin temperature clearly rises above the initial skin temperature (for details see item 4 'Measurement of temperature of the skin').

Subsequently, the skin became noticeably warm, which could also be determined by touching it with the hand. Furthermore, in case of a caucasian skin, a clear redness of the skin developed, wherein the redness was visually detectable to the naked eye.

### 11. Determination of joint condition - tender joints (TJ) and swollen joints (SJ)

The investigation and determination of the joint condition has been performed by a physician or rheumatologist. A tender joint (TJ) is pressure sensitive but not necessarily swollen. A swollen joint (SJ) shows a swelling, which is palpable by manual investigation and/or visually detectable to the naked eye. The amount of TJ and SJ is counted. A reduction in the amounts of TJ and/or SJ, respectively, is an indication for a reduction in inflammation, mitigation of the disease and an improvement of the patient's health condition.

### 12. CRP - C-reactive Protein

The CRP (C-reactive protein) is the most important blood laboratory value for detecting and monitoring inflammation in the body. It was determined by a certified laboratory. A low CRP indicates the absence of an inflammation, while a high CRP is indicative for an inflammation to be present.

It is a highly sensitive marker and already a tiny focus of inflammation may cause a rise of the CRP, however, a low CRP is a clear indication for lack of any inflammations in the patient's body.

**Table 1: Usual scoring of CRP-ranges for an adult human:**

| ***Score*** | ***CRP [mg*/*dl {milligram*/*decilitre (10 ml)}]*** |
|---|---|
| normal | 1 |
| slightly increased | >1 to 4 |
| moderately increased | >4 to 20 |
| strongly increased | >20 |

### 13. BASDAI - Bath Ankylosing Spondylitis Disease Activity Index

The BASDAI (Bath Ankylosing Spondylitis Disease Activity Index) is a validated diagnostic test which allows a physician, usually a rheumatologist, to determine the effectiveness of a current drug therapy, or the need to institute a new drug therapy for the treatment of ankylosing spondylitis (Morbus Bechterew). A higher BASDAI indicates a higher level of suffering pressure and suboptimal disease control in the patient, while a lower BASDAI indicates an improved life quality of the patient due to better disease control.

The BASDAI consists of a 0 to 10 scale measuring discomfort, pain, and fatigue (0 being no problem and 10 being the worst problem) in response to six questions asked of the patient pertaining to the five major symptoms of ankylosing spondylitis:
*1) Fatigue;*
*2) Spinal pain;*
*3) Arthralgia (joint pain; tender joints) or swelling;*
*4) Enthesitis, or inflammation of tendons and ligaments (areas of localized tenderness where connective tissues insert into bone);*
*5) Morning stiffness duration;*
*6) Morning stiffness severity.*

To give each symptom equal weighting, the average of the two scores relating to morning stiffness [5*)* and *6)*]) is taken. The resulting 0 to 50 score is divided by 5 to give a final 0 to 10 BASDAI score. Scores of 4 or greater suggest suboptimal control of disease. Hence, those patients are good candidates for testing the method according to present invention.

### EXAMPLES

All patient were selected solely based on their diagnosis without performing a preselection in view of other parameters. Furthermore, all patients treated according to the present invention reported of an onset of beneficial effects within 12 h to 24 h after receiving the treatment.

### Example 1: Rheumatoid arthritis

### 1000 IU IFN-γ (high dose) and 200 IU IL-2 subcutaneously plus heat cream

Patient 01 was a 46 years old female, 58 kg, suffering from rheumatoid arthritis of M05.80 in accordance with the standard ICD-10-GM 2021 for over 20 years. She showed swelling of joints (SJ) and joint pain (TJ) as indicated in Table 2. She was not able to walk down stairs forwardly, could not sleep through and was strongly impaired in daily life. The patient described her condition as strongly affected by the disease.

Over the course of the treatment, she received a constant basis treatment of 30 mg cortisone per day. The dose, composition and frequency of the cortisone basis treatment remained constant over the time of the treatment.

In preparation for the treatment, 100 µl injection solution containing 200 IU IL-2 and 100 µl injection solution containing 1000 IU IFN-γ (high IFN-γ dose) were combined in order to prepare a combined injection solution.

The treatment was performed by administering topically on the skin of the upper side of the forearm a heat cream, Kytta^{®} heat balm (see also item 10 'Vasodilation // increased blood flow // increased the skin temperature // redness of the skin by using heat cream). 50 mg to 800 mg Kytta^{®} heat balm was administered to a skin area of about 25 cm². After the skin has turned red and warm and an increase in the skin temperature was palpable after about 15 min, the combined injection solution was subcutaneously injected within the red and warmed skin area. The treatment regimen was performed as indicated in Table 2.

After about 1.5 weeks, according to the patient statement, pain and fatigue were significantly reduced. The swelling in the joints and the tenderness/pain of the joints was remarkably reduced, the patient was able to walk down stairs forwardly, could sleep through and was less impaired in daily life. The CRP decreased remarkably. Details and results are indicated in Table 2 and Figs. 1, 2 and 3. The patient then described her condition as good.

Furthermore, in patient 01 no treatment over weeks 7 to 12 has been performed, It can be seen from Table 2 and Fig. 1, that after 6 weeks without treatment the CRP returned to 5, that is the initial value of week 1 before starting the treatment.

Furthermore, in patient 01, placebo (week 13) and incorrect treatment (weeks 14 to 17) were performed, which is described in Comparative Examples 1 and 3 and as indicated in Table 2 and Figs. 1, 2 and 3.

### Example 2: Rheumatoid arthritis

### 100 IU IFN-γ (low dose) and 200 IU IL-2 subcutaneously plus heat pad

Patient 02, was a 59 years old female, 62 kg, suffering from rheumatoid arthritis of M05.80 in accordance with the standard ICD-10-GM 2021 for over 20 years. She showed deformations and swelling of joints (SJ) and joint pain (TJ), particularly, in both hands (all ten proximal interphalangeal and methacarophalangia were affected) as indicated in Table 2. The patient was not able to grab properly and opening of bottles. The patient described her condition as strongly affected by the disease.

Over the course of the treatment, she was intended to receive a constant basis treatment of 15 mg Methotrexat (MTX) per week. However, after the second treatment, she forgot to inject the MTX. Her condition and pain has improved to such an extent that she was no longer in need of the MTX.

In preparation for the treatment, 100 µl injection solution containing 200 IU IL-2 and 100 µl injection solution containing 100 IU IFN-γ (low IFN-γ dose) were combined in order to prepare a combined injection solution.

Treatment was performed by applying topically a heat pad on the upper side of the forearm as described above under item 9 'Vasodilation // increased blood flow // increased the skin temperature // redness of the skin by using a heat pad'. The heat pad was applied for about 2 min upon the skin has turned red and warm and an increase in the skin temperature was palpable. About 1 min after removing the heat pad, the combined injection solution was subcutaneously injected within the red and warmed skin area. The treatment regimen was performed as indicated in Table 2.

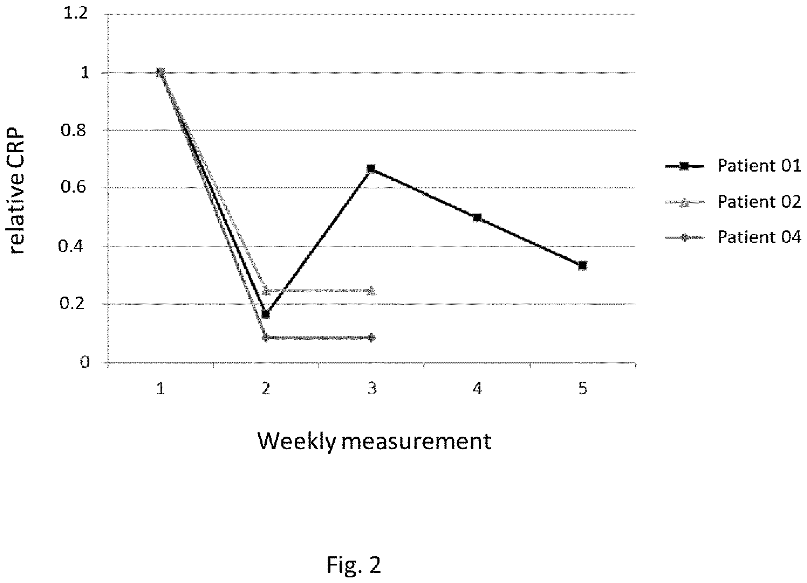

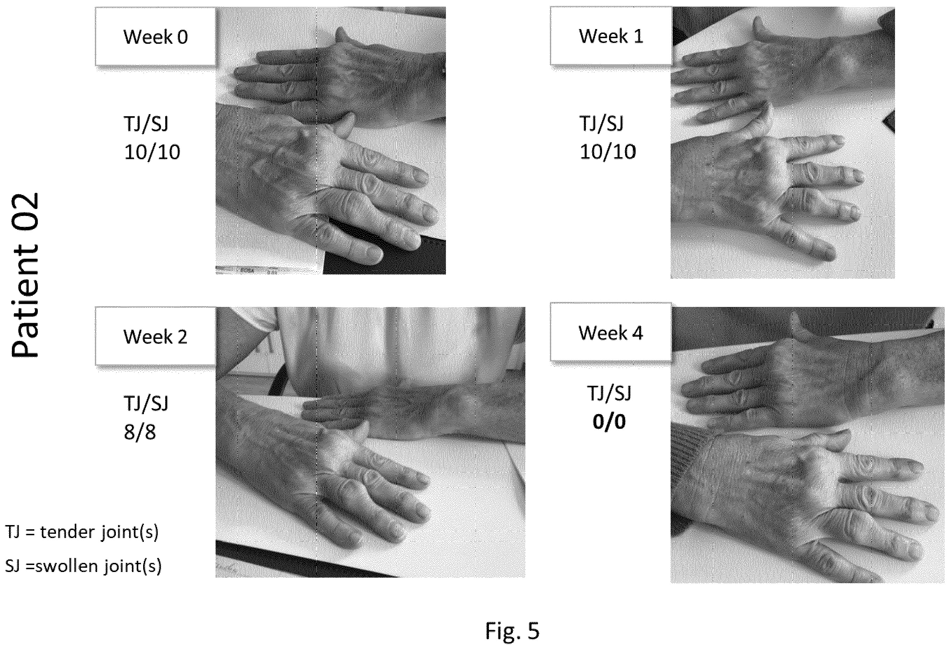

As already stated above, after the second treatment the patient's condition had improved that much that she forgot to inject the MTX. Furthermore, the patient reported that the fatigue was reduced. The CRP decreased remarkably and did not rise even after 4 weeks without treatment (weeks 4 to 7) as indicated in Table 2 and Figs. 2 and 3. After 4 weeks of continuous treatment (weeks 8 to 11) or after 12 weeks in total with interruptions (weeks 1 to 11), respectively, the tender and swollen joints (10/10) in both hands was reduced to zero (0/0), see Table 2 and Fig. 5.

### Example 3: Rheumatoid arthritis

### 100 IU IFN-γ (low dose) and 200 IU IL-2 subcutaneously or without IL-2 plus heat pad

Patient 03 was a 83 years old female, 78 kg, suffering from rheumatoid arthritis of M05.80 in accordance with the standard ICD-10-GM 2021 for over 45 years. She showed deformations and swelling of joints and tender joints in both shoulders, knees and hands including all ten proximal interphalangeal and methacarophalangia. The patient was not able to walk down stairs forwardly, perform gardening and was strongly impaired in housekeeping. The patient described her condition as strongly affected by the disease.

Over the course of the treatment, she received a constant basis treatment with pain killers. The dose, composition and frequency of the pain killer basis treatment remained constant over the time of the treatment.

In preparation for the treatment, 100 µl injection solution containing 200 IU IL-2 and 100 µl injection solution containing 100 IU IFN-γ (low IFN-γ dose) were combined in order to prepare a combined injection solution.

Treatment was performed by applying topically a heat pad on the upper side of the forearm as described above under item 9 'Vasodilation // increased blood flow // increased the skin temperature // redness of the skin by using a heat pad'. The heat pad was applied for about 2 min upon the skin has turned red and warm and an increase in the skin temperature was palpable. About 1 min after removing the heat pad, 100 IU IFN-γ (without IL-2) were subcutaneously injected (weeks 2 to 7) or the combined injection solution was subcutaneously injected (weeks 9 to 12) within the red and warmed skin area. The treatment regimen was performed as indicated in Table 2.

As can be seen from Table 2, the low concentration of 100 IU IFN-γ without IL-2 (weeks 2 to 7) did not have any improving effect on the joint condition. The CRP is initially already quite low. The patient 03 reported to be free of pain apart from lumbago and reduced fatigue for about 3 to 4 days after treatment, but not over the entire weeks. However, when the low concentration of 100 IU IFN-γ was combined with IL-2 by subcutaneously injecting the combined injection solution, the patient reported, that the pain free condition apart from lumbago and reduced fatigue was prolonged over the entire week until the next treatment. Hence, IL-2 synergistically improved the effect of IFN-γ, while the administration of IL-2 does not provide any effect as can be seen from Comparative Example 2.

Furthermore, it can be seen from Table 2, that in week 9 the CRP has strongly increased to 7.1. As already stated above, the CRP is a highly sensitive marker. It is annotated, that the CRP in this case is increased due to an inflammation in a big toe joint resulting from mechanical overstrain, which is not related to rheumatoid arthritis. Hence, the CRP increase is presumably not related to rheumatoid arthritis and the decrease CRP may not be related to the treatment but rather to a healing of the overstrain.

Furthermore, in patient 03, placebo (week 1) were performed, which is described in Comparative Examples 2 and as indicated in Table 2 and Figs. 1, 2 and 3.

### Example 4: Rheumatoide arthritis

### 1000 IU IFN-γ (high dose) and 200 IU IL-2 subcutaneously plus heat pad

Patient 04, was a 49 years old female, 55 kg, suffering from rheumatoid arthritis of M05.80 in accordance with the standard ICD-10-GM 2021 for about 35 years. Her rheumatoid condition was already very advanced.

She showed strong deformations of her joints and has received already several joint replacements (>10). Furthermore, she showed swelling of joints (SJ) and joint pain (TJ) as indicated in Table 2. The patient described her condition as strongly affected by the disease.

Over the course of the treatment, she was intended to receive a constant basis treatment of a combination of several medicaments. The dose, composition and frequency of the basis treatment remained constant over the time of the treatment.

In preparation for the treatment, 100 µl injection solution containing 200 IU IL-2 and 100 µl injection solution containing 1000 IU IFN-γ (high IFN-γ dose) were combined in order to prepare a combined injection solution. The treatment regimen was performed as indicated in Table 2.

In weeks 1 to 3, the treatment was performed by administering topically on the skin of the upper side of the forearm heat cream, Kytta^{®} heat balm (see also item 10 'Vasodilation // increased blood flow // increased the skin temperature // redness of the skin by using heat cream). 50 mg to 800 mg Kytta^{®} heat balm was administered to a skin area of about 50 cm². After about 6 min, the combined injection solution was subcutaneously injected within the area of the skin the heat cream was applied. As can be seen from Table 2 and Figs. 2 and 3 that the CRP after the first treatment (week 2) reduced to a remarkably low value. Furthermore, the tender and swollen joints were strongly reduced from 14/11 (TJ/SJ) to 3/2 (TJ/SJ).

In this regards it is pointed out that the skin temperature is 6 min after application of the heat cream even lower than the initial skin temperature (see item 4 'Measurement of temperature of the skin'), however, after 15 min, the temperature strongly increases, that is after the administration the IFN-γ and IL-2. This indicates, that a vasodilation is also sufficient to establish after injecting IFN-γ and IL-2 in order to achieve the technical effect.

In weeks 6, 8 and 10, the treatment was performed by applying topically a heat pad on the upper side of the forearm as described above under item 9 'Vasodilation // increased blood flow // increased the skin temperature // redness of the skin by using a heat pad'. The heat pad was applied for about 5 min upon the skin has turned red and warm and an increase in the skin temperature was palpable. About 1 min after removing the heat pad, the combined injection solution was subcutaneously injected within the red and warmed skin area. The treatment regimen was performed as indicated in Table 2.

As can be seen from Table 2 and Figs. 2 and 3 that the CRP after the first treatment (week 2) reduced and remained low even with a two week interval of treatment. Similarly, the tender and swollen joints remained strongly reduced from 14/11 (TJ/SJ) to 3/2 (TJ/SJ).

However, the pain was not improved over the entire trial, which was to be expected. The degeneration of her joint was already very advanced. Hence, the pain resulted from arthrosis and joint degeneration and was not primarily due to joint inflammation. Obviously, it cannot be expected that the immunomodulatory method according to the present invention will improved or restore mechanically destructed joints.

### Example 5: Polyarthritis

### 100 IU IFN-γ (low dose) subcutaneously plus heat pad

Patient 05, was a 77 years old female, 58 kg, suffering from polyarthritis of unknown origin of M25.5 in accordance with the standard ICD-10-GM 2021 for over 2 years. She showed swelling of shoulder joints and was not able to lift her arm over her head. The patient described her condition as strongly affected by the disease and as highly painful.

Over the course of the treatment, she received a constant basis treatment with pain killers. The dose, composition and frequency of the pain killer basis treatment remained constant over the time of the treatment.

In preparation for the treatment, 100 µl injection solution containing 200 IU IL-2 and 100 µl injection solution containing 100 IU IFN-γ (low IFN-γ dose) were combined in order to prepare a combined injection solution.

Treatment was performed by applying topically a heat pad on the upper side of the forearm as described above under item 9 'Vasodilation // increased blood flow // increased the skin temperature // redness of the skin by using a heat pad'. The heat pad was applied for about 2 min upon the skin has turned red and warm and an increase in the skin temperature was palpable. About 1 min after removing the heat pad, 100 IU IFN-γ (without IL-2) were subcutaneously injected (weeks 2 to 7) or the combined injection solution was subcutaneously injected (weeks 9 to 12) within the red and warmed skin area. The treatment regimen was performed as indicated in Table 2.

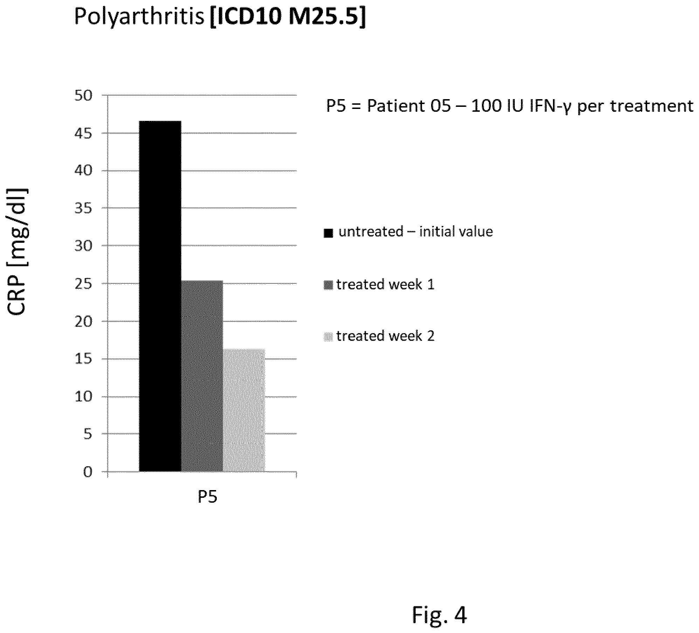

After the second treatment (week 2), the patient was able to lift her hands over the head and the disease condition was strongly reduced. Furthermore, as can be seen from Table 2 and Fig. 4, the CRP was reduced to almost a third of the initial CRP, and the amount of tender joints was reduced from 8 to 3.

### Example 6: Ankylosing spondylitis (Morbus Bechterew)

### 1000 IU IFN-γ (high dose) and 200 IU IL-2 subcutaneously plus heat cream or heat pad

Patient 06 was a 61 years old female, 86 kg, suffering from ankylosing spondylitis. She has strong pain, particularly in the back, was suffering from fatigue and was not able to work though for more than 4 days. The patient described her condition as strongly affected by the disease.

Over the course of the treatment, she was intended to receive a constant basis treatment of a combination of several medicaments. The dose, composition and frequency of the basis treatment remained constant over the time of the treatment.

In preparation for the treatment, 100 µl injection solution containing 200 IU IL-2 and 100 µl injection solution containing 1000 IU IFN-γ (high IFN-γ dose) were combined in order to prepare a combined injection solution. The treatment regimen was performed as indicated in Table 2.

In weeks 1 to 4, the treatment was performed by administering topically on the skin of the upper side of the forearm heat cream, Kytta^{®} heat balm (see also item 10 'Vasodilation // increased blood flow // increased the skin temperature // redness of the skin by using heat cream). 50 mg to 800 mg Kytta^{®} heat balm was administered to a skin area of about 25 cm². After about 8 min, the combined injection solution was subcutaneously injected within the area of the skin the heat cream was applied. The patient reported about a clear improvement in pain that after treatment for about 4 days. Furthermore, the fatigue war reduced and she was able to work 2 weeks trough.

In this regards it is pointed out that the skin temperature is 8 min after application of the heat cream even lower than the initial skin temperature (see item 4 'Measurement of temperature of the skin'), however, after 15 min, the temperature strongly increases, that is after the administration the IFN-γ and IL-2. This indicates, that a vasodilation is also sufficient to establish after injecting IFN-γ and IL-2 in order to achieve the technical effect.

In weeks 7, 9 and 11, the treatment was performed by applying topically a heat pad on the upper side of the forearm as described above under item 9 'Vasodilation // increased blood flow // increased the skin temperature // redness of the skin by using a heat pad'. The heat pad was applied for about 5 min upon the skin has turned red and warm and an increase in the skin temperature was palpable. About 1 min after removing the heat pad, the combined injection solution was subcutaneously injected within the red and warmed skin area. The treatment regimen was performed as indicated in Table 2. Again, the patient reported about a clear improvement in pain that after treatment for about 4 days. Furthermore, the fatigue war reduced and she was able to work 2 weeks trough.

Furthermore, the BASDAI was determined. Under treatment in week 11, the BASDAI was 4.6, which approaches a good control of the disease indicated by a BASDAI of 4 or less (see above item 13 'BASDAI').

Furthermore, with heat cream and heat pad, the amount of tender and swollen joints was reduced.

After finishing the trail, the patient insistently requested that the treatment be continued. However, since some of the effects, particularly the pain, lasted only for about 4 days after treatment, this suggests that the frequency of the treatment might be too low and should be increased to, for instance, two treatments per week.

### Example 7: Rheumatoide arthritis

### Injection of PBMCs

Patient 07 was a 79 years old female, 75 kg, suffering from rheumatoid arthritis of M05.80 in accordance with the standard ICD-10-GM 2021 for over 40 years. She showed deformations and swelling of joints and joint pain in both shoulders, knees and hands (all ten proximal interphalangeal and methacarophalangia). She has and is still treated with pain killers. The patient was not able to walk down stairs forwardly, perform gardening and needle work and was strongly impaired in housekeeping. The patient described her condition as strongly affected by the disease.

The dose and composition of the pain killer remained constant over the time of the treatment with PBMCs.

PBMCs were prepared as described under 'Preparation of PBMCs' and administered every two weeks in a single administration dose subcutaneously in an amount of 9×10⁶ PBMCs// 4.5×10⁶ PBMCs, prepared in a volume of 200 µl 0.9 % NaCl.

100 µl injection solution containing 200 IU IL-2 and 100 µl injection solution containing 100 IU IFN-γ were combined (i) and subcutaneously injected 45 min after the injection of the PBMCs in a vicinity of 1 cm or less around the injection site of the PBMCs, wherein the patient performed the injection as a self-treatment.

Alternatively, 100 µl injection solution containing 200 IU IL-2 and 100 µl injection solution containing 1000 IU IFN-γ were combined (ii) and subcutaneously 45 min after the injection of the PBMCs injected in a vicinity of 1 cm or less around the injection site of the PBMCs, wherein the patient performed the injection as a self-treatment.

After about two day for both (i) and about one day for (ii), according to the patient statement, the pain was significantly reduced to zero and the dosage of the pain killers administered was halved. The swelling in the joint was remarkably reduced, the patient was able to walk down stairs forwardly, perform gardening and needle work and the impairment in housekeeping was strongly reduced. The patient described her condition as good.

The effect lasted for both (i) and (ii) for about 10 days each.

### Comparative Example 1

### Negative control/Placebo: using vasodilation plus IL-2 without IFN-γ or IFN-γ (high amount of 1000 IU) alone without vasodilation

Furthermore, patient 01 of Example 1 experienced a treatment according to a negative control.

For that reason, on the upper side of the right forearm, treatment with heat creme as described in Example 1 was performed. After the skin has turned red and warm and an increase in the skin temperature was palpable after about 15 min, 100 µl injection solution containing 200 IU IL-2 were subcutaneously injected within the red and warmed skin area.

Furthermore, on the upper side of the left forearm, 100 µl injection solution containing 1000 IU IFN-γ were injected subcutaneously. On the left arm, no vasodilation was caused and/or no IL-2 was administered.

From Table 1 (grey highlighted) it can be seen that neither the CRP nor the joint condition have improved in week 14. Hence, neither vasodilation nor IL-2 nor the combination of both cause the technical effect. Additionally, also the patient did not report about any health improvements or relieves from the disease. Furthermore, also the administration of 1000 IFN-γ alone without vasodilation cannot cause the technical effect.

### Comparative Example 2

### Negative control/Placebo: using vasodilation plus IL-2 without IFN-γ or IFN-γ (low amount of 100 IU) alone without vasodilation

Patient 03 of Example 3 experienced a treatment according to a negative control.

For that reason, on the upper side of the right forearm, treatment with a head pad as described in Example 3 was performed. About 1 min after removing the heat pad, 100 IU IL-2 were subcutaneously injected.

Furthermore, on the upper side of the left forearm, 100 µl injection solution containing 100 IU IFN-γ were injected subcutaneously. On the left arm, no vasodilation was caused and/or no IL-2 was administered.

From Table 1 (grey highlighted) it can be seen that neither the CRP nor the joint condition have improved in week 2. Hence, neither vasodilation nor IL-2 nor the combination of both cause the technical effect. Additionally, also the patient did not report about any health improvements or relieves from the disease. Furthermore, also the administration of 100 IU IFN-γ alone without vasodilation cannot cause the technical effect.

### Comparative Example 3

### Erroneous treatment using heat balm plus IL-2 without IFN-γ

Furthermore, in patient 01 of Example 1 the treatment was performed over 4 weeks incorrectly, however, the patient as well as the physician believed the conduction was correct. Therefore, a placebo effect could be excluded.

The erroneous treatment consisted in the fact that the heat creme was applied correctly as described in Example 1, however, the injection of the combined injection solution was performed more than 1.5 h after application of the heat creme.

From Table 1 (grey highlighted) it can be seen that neither the CRP nor the joint condition improved during weeks 14 to 18. As can be seen from item 4 'Measurement of the temperature of the skin' 1.25 h after application of the heat creme, the skin temperature started to return to the initial value. Hence, without warming the technical effect cannot be achieved. The combination of IFN-γ and IL-2 in the indicated concentration is not sufficient.

### Comparative Example 4

### Topical administration of extremely high amount of IFN-γ in the vicinity of an inflamed joint

Patient 08 was a 45 years old female, 65 kg, suffering from arthritis in the knees. She showed swelling of joints and joint pain in both knees. She was untreated with pain killers or any other medicamentation. The patient was hardly able to walk down stairs forwardly under great pain. The patient described her condition as strongly affected since neither long walks nor sportive activities were possible.

A total amount of IFN-γ 2×10⁴ IU was administered twice daily to each inflamed knee topically. Hence, an extremely high amount of IFN-γ was administered close to a site of inflammation. A vasodilation was not induced. The condition was worsening over the treatment for three days, then the treatment was stopped.

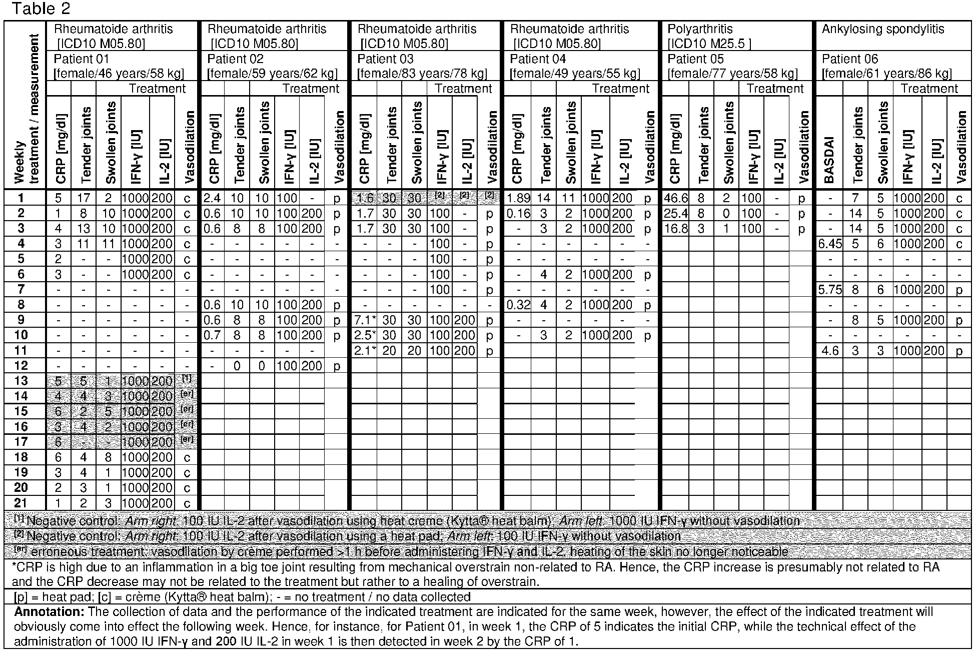

## Claims

1. An immunomodulatory substance for use in a method for treating and/or preventing an inflammatory disease, immunological disease and/or autoimmunological disease, wherein the method comprises the steps of:
(A) generating an accumulation of PBMCs within the skin of a subject; and
(B) administering the immunomodulatory substance to the skin of said subject.

2. A method for treating and/or preventing an inflammatory disease, immunological disease and/or autoimmunological disease, wherein the method comprises the steps of:
(A) generating an accumulation of PBMCs within the skin of a subject; and
(B) administering the immunomodulatory substance to the skin of said subject.

3. A topical dosage form comprising an immunomodulatory substance; and/or
- a dosage form suitable for injection comprising an immunomodulatory substance;
preferably in a method for treating and/or preventing an inflammatory disease, immunological disease and/or autoimmunological disease, wherein the method comprises the steps of:
(A)generating an accumulation of PBMCs within the skin of a subject; and
(B)administering the immunomodulatory substance to the skin of said subject, by applying the topical dosage form to the skin and/or by performing an injection into the skin by using the dosage form suitable for injection.

4. A first immunomodulatory substance; and
- a second immunomodulatory substance;
for use in a method for treating and/or preventing an inflammatory disease, immunological disease and/or autoimmunological disease, wherein the method comprises the steps of:
(A) generating an accumulation of PBMCs within the skin of a subject;
(B) administering the first immunomodulatory substance to the skin of said subject; and
(C) administering the second immunomodulatory substance to the skin of said subject.

5. A method for treating and/or preventing an inflammatory disease, immunological disease and/or autoimmunological disease, wherein the method comprises the steps of:
(A) generating an accumulation of PBMCs within the skin of a subject;
(B) administering the first immunomodulatory substance to the skin of said subject; and
(C) administering the second immunomodulatory substance to the skin of said subject.

6. A topical dosage form comprising a first immunomodulatory substance; and/or
a topical dosage form comprising a second immunomodulatory substance; and/or
- a topical dosage form comprising the first immunomodulatory substance and second immunomodulatory substance; and/or
- a dosage form suitable for injection comprising the first immunomodulatory substance; and/or
- a dosage form suitable for injection comprising the second immunomodulatory substance, and/or
- a dosage form suitable for injection comprising the first immunomodulatory substance and the second immunomodulatory substance,
preferably in a method for treating and/or preventing inflammatory diseases, immunological diseases and autoimmunological diseases, immunological disease and/or autoimmunological diseases in any one of the embodiments as described herein, preferably in a method wherein the method comprises the steps of:
(A) generating an accumulation of PBMCs within skin of a subject;
(B) applying the first immunomodulatory substance to the skin of the subject by applying one of the topical dosage forms comprising the first immunomodulatory substance to the skin and/or by performing an injection into the skin by using one of the dosage form suitable for injection comprising the first immunomodulatory substance; and
(C) applying the second immunomodulatory substance to the skin of the subject by applying one of the topical dosage forms comprising the second immunomodulatory substance to the skin and/or by performing an injection into the skin by using one of the dosage form suitable for injection comprising the second immunomodulatory substance.

7. An immunomodulatory substance for use in a method for treating and/or preventing an inflammatory disease, immunological disease and/or autoimmunological disease, wherein the method comprises the steps of:
(A-1) generating a vasodilation of the capillaries within the skin of a subject; and
(B) administering the immunomodulatory substance to the skin of said subject.

8. A method for treating and/or preventing an inflammatory disease, immunological disease and/or autoimmunological disease, wherein the method comprises the steps of:
(A-1) generating a vasodilation of the capillaries within the skin of a subject; and
(B) administering the immunomodulatory substance to the skin of said subject.

9. An immunomodulatory substance for use in a method for treating and/or preventing an inflammatory disease, immunological disease and/or autoimmunological disease, wherein the method comprises the steps of:
(A-2) generating an increased blood flow within the skin of a subject, wherein the blood flow is preferably increased by 1 % or more, preferably when measured as described in the methods measurement of the blood flow, and/or is increased by 10 µl/s or more, preferably when measured as described in the methods measurement of the blood flow; and
(B) administering the immunomodulatory substance to the skin of said subject.

10. A method for treating and/or preventing an inflammatory disease, immunological disease and/or autoimmunological disease, wherein the method comprises the steps of:
(A-2) generating an increased blood flow within the skin of a subject, wherein the blood flow is preferably increased by 1 % or more, preferably when measured as described in the methods measurement of the blood flow, and/or is increased by 10 µl/s or more, preferably when measured as described in the methods measurement of the blood flow; and
(B) administering the immunomodulatory substance to the skin of said subject.

11. An immunomodulatory substance for use in a method for treating and/or preventing an inflammatory disease, immunological disease and/or autoimmunological disease, wherein the method comprises the steps of:
(A-3) generating an increased temperature on the skin of a subject, wherein preferably the temperature is increased by 1 % or more and/or is increased by 0.2 °C or more, preferably when measured as described in the methods measurement of the temperature of the skin; and
(B) administering the immunomodulatory substance to the skin of said subject.

12. A method for treating and/or preventing an inflammatory disease, immunological disease and/or autoimmunological disease, wherein the method comprises the steps of:
(A-3) generating an increased temperature on the skin of a subject, wherein preferably the temperature is increased by 1 % or more and/or is increased by 0.2 °C or more, preferably when measured as described in the methods measurement of the temperature of the skin; and
(B) administering the immunomodulatory substance to the skin of said subject;

13. An immunomodulatory substance for use in a method for treating and/or preventing an inflammatory disease, immunological disease and/or autoimmunological disease, wherein the method comprises the steps of:
(A-4) generating a redness on the skin of a subject, wherein the redness is visually detectable to the naked eye; and
(B) administering the immunomodulatory substance to the skin of said subject.

14. A method for treating and/or preventing an inflammatory disease, immunological disease and/or autoimmunological disease, wherein the method comprises the steps of:
(A-4) generating a redness on the skin of a subject, wherein the redness is visually detectable to the naked eye; and
(B) administering the immunomodulatory substance to the skin of said subject.

15. An immunomodulatory substance for use in a method for treating and/or preventing an inflammatory disease, immunological disease and/or autoimmunological disease, wherein the method comprises the steps of:
(A-5) applying heat to the skin of a subject; and
(B) administering the immunomodulatory substance to the skin of said subject.

16. A method for treating and/or preventing an inflammatory disease, immunological disease and/or autoimmunological disease, wherein the method comprises the steps of:
(A-5) applying heat to the skin of a subject; and
(B) administering the immunomodulatory substance to the skin of said subject.

17. An immunomodulatory substance for use in a method for treating and/or preventing an inflammatory disease, immunological disease and/or autoimmunological disease, wherein the method comprises the steps of:
(A-6) applying mechanical agitation and/or vibration to the skin of a subject; and
(B) administering the immunomodulatory substance to the skin of said subject.

18. A method for treating and/or preventing an inflammatory disease, immunological disease and/or autoimmunological disease, wherein the method comprises the steps of:
(A-6) applying mechanical agitation and/or vibration to the skin of a subject; and
(B) administering the immunomodulatory substance to the skin of said subject.

19. An immunomodulatory substance for use in a method for treating and/or preventing an inflammatory disease, immunological disease and/or autoimmunological disease, wherein the method comprises the steps of:
(A-7) applying negative pressure to the skin of a subject; and
(B) administering the immunomodulatory substance to the skin of said subject.

20. A method for treating and/or preventing an inflammatory disease, immunological disease and/or autoimmunological disease, wherein the method comprises the steps of:
(A-7) applying negative pressure to the skin of a subject; and
(B) administering the immunomodulatory substance to the skin of said subject.

21. An immunomodulatory substance for use in a method for treating and/or preventing an inflammatory disease, immunological disease and/or autoimmunological disease, wherein the method comprises the steps of:
(A-8) administering a blood-circulation-increasing agent to the skin of a subject; and
(B) administering the immunomodulatory substance to the skin of said subject.

22. A method for treating and/or preventing an inflammatory disease, immunological disease and/or autoimmunological disease, wherein the method comprises the steps of:
(A-8) administering a blood-circulation-increasing agent to the skin of a subject; and
(B) administering the immunomodulatory substance to the skin of said subject.

23. An immunomodulatory substance for use in a method for treating and/or preventing an inflammatory disease, immunological disease and/or autoimmunological disease, wherein the method comprises the steps of:
(A-9) administering a vasodilating agent to the skin of a subject; and
(B) administering the immunomodulatory substance to the skin of said subject.

24. A method for treating and/or preventing an inflammatory disease, immunological disease and/or autoimmunological disease, wherein the method comprises the steps of:
(A-9) administering a vasodilating agent to the skin of a subject; and
(B) administering the immunomodulatory substance to the skin of said subject.

25. An immunomodulatory substance for use in a method for treating and/or preventing an inflammatory disease, immunological disease and/or autoimmunological disease, wherein the method comprises the steps of:
(A-10) injecting PBMCs into the skin of a subject, preferably the skin of the skin area; and
(B) administering the immunomodulatory substance to the skin of said subject.

26. A method for treating and/or preventing an inflammatory disease, immunological disease and/or autoimmunological disease, wherein the method comprises the steps of:
(A-10) injecting PBMCs into the skin of a subject, preferably the skin of the skin area; and
(B) administering the immunomodulatory substance to the skin of said subject;

27. Immunomodulatory substance for use in a method or method according to any one of the preceding claims wherein the method further comprises the step of:
(C) administering a second immunomodulatory substance to the skin.

28. Immunomodulatory substance for use in a method or method according to claim 27 wherein the second immunomodulatory substance administered in step (C) comprises or consists of IL-2.

29. Immunomodulatory substance for use in a method or method according to any one of the preceding claims wherein the PBMCs are lymphocytes, such as T-cells, more preferably are naïve lymphocytes, and most preferably are naïve T-cells.

30. Immunomodulatory substance for use in a method or method according to any one of the preceding claims wherein in step (A) the accumulation of PBMCs is generated by:
(A-1) generating a vasodilation of the capillaries within the skin; and/or
(A-2) generating an increased blood flow within the skin, wherein the blood flow is increased by 1% or more, preferably when measured as described in the methods, item 3 'measurement of the blood flow' and/or is increased by 10 µl/s or more, preferably when measured as described in the methods, item 3 'measurement of the blood flow' and/or
(A-3) generating an increased temperature on the skin, wherein preferably the temperature is increased by 1 % or more and/or is increased by 0.2 °C or more, preferably when measured as described in the methods 'measurement of the temperature of the skin'; and/or
(A-4) generating a redness on the skin, wherein the redness is visually detectable to the naked eye; and/or
(A-5) applying heat to the skin; and/or
(A-6) applying mechanical agitation to the skin; and/or
(A-7) applying negative pressure to the skin; and/or
(A-8) administering a blood-circulation-increasing agent to the skin; and/or
(A-9) administering a vasodilating agent to the skin; and/or
(A-10) injecting PBMCs into the skin of a subject, preferably the skin of the skin area;

31. Immunomodulatory substance for use in a method or method according to any one of the preceding claims wherein the immunomodulatory substance administered in step (B) comprises, or consists of, IFN-γ.

32. An immunomodulatory substance for use in a method for treating and/or preventing an inflammatory diseases, immunological disease and/or autoimmunological disease, wherein the method comprises the steps of:
II. administering the immunomodulatory substance to the skin of a subject; wherein the amount administered does not cause an systemic increase of the concentration of the immunomodulatory substance of the subject.

33. A method for treating and/or preventing an inflammatory disease, immunological disease and/or autoimmunological disease, wherein the method comprises the steps of:
I. administering an immunomodulatory substance to the skin of a subject; wherein the amount administered does not cause an systemic increase of the concentration of the immunomodulatory substance of the subject.

34. An immunomodulatory substance for use in a method for treating and/or preventing an inflammatory diseases, immunological disease and/or autoimmunological disease, wherein the method comprises the steps of:
III. administering the immunomodulatory substance to the skin of a subject; wherein the amount administered does not cause an systemic increase of the concentration of the immunomodulatory substance of the subject; and
IV. administering the second immunomodulatory substance to the skin of a subject; wherein the amount administered does not cause an systemic increase of the concentration of the immunomodulatory substance of the subject.

35. A method for treating and/or preventing an inflammatory diseases, immunological disease and/or autoimmunological disease, wherein the method comprises the steps of:
I. administering a immunomodulatory substance to the skin of a subject; wherein the amount administered does not cause an systemic increase of the concentration of the immunomodulatory substance of the subject; and
II. administering a second immunomodulatory substance to the skin of a subject; wherein the amount administered does not cause an systemic increase of the concentration of the immunomodulatory substance of the subject.

36. An immunomodulatory substance for use in a method for treating and/or preventing an inflammatory diseases, immunological disease and/or autoimmunological disease, wherein the method comprises the steps of:
II. topically administering IFN-γ to the skin area of a subject distanced to a site of immunological activity or challenge.

37. A method for treating and/or preventing an inflammatory diseases, immunological disease and/or autoimmunological disease, wherein the method comprises the steps of:
I. topically administering IFN-γ to the skin area of a subject distanced to a site of immunological activity or challenge.

38. A kit of parts comprising
i) a means for generating an accumulation of PBMCs within the skin of a subject and
ii) an immunomodulatory substance.

39. A kit of parts comprising
i) an immunomodulatory substance, preferably IFN-γ and
ii) a second immunomodulatory substance, preferably IL-2.

40. A plaster, pad or bandage comprising IFN-γ.

41. A cartridge for an injection pen, carpule and/or syringe comprising IFN-γ.

42. A composition for topical application, comprising IFN-γ and a blood-circulation-increasing substance.

43. A composition for topical application, comprising IFN-γ and IL-2.

44. A composition for topical application and/or injection, comprising IFN-γ in an amount of 50.000 IU/ml or less or 2.500 ng/ml or less.
